# EUROPÄISCHE PATENTSCHRIFT

(11) **EP 3 793 401 B1**
(45) Veröffentlichungstag und Bekanntmachung des Hinweises auf die Patenterteilung: **26.02.2025**
(21) Anmeldenummer: 19720545.3
(22) Anmeldetag: 26.04.2019
(51) Int. Cl.: G01N 21/359, A61B 5/00, G01N 21/27, G01N 21/3563, A45D 44/00

(54) **BESTIMMUNG EINES DEHNUNGSGRADES VON HAAR MIT EINEM NIR-SENSOR**
DETERMINATION OF A DEGREE OF ELONGATION OF HAIR USING A NIR SENSOR
DÉTERMINATION D'UN TAUX D'ALLONGEMENT DU CHEVEU AVEC UN CAPTEUR PROCHE INFRAROUGE

(30) Priorität: 16.05.2018 DE 102018207557
(43) Veröffentlichungstag der Anmeldung: 24.03.2021
(73) Patentinhaber: Henkel AG & Co. KGaA, 40589 Düsseldorf (DE)
(72) Erfinder: KNUEBEL, Hans Georg, 40219 Düsseldorf (DE); BONNIN, Lucile, 10437 Berlin (DE); KROOS, Astrid, 40789 Monheim (DE); KOENEN, Annika, 41516 Grevenbroich (DE); SCHULZE ZUR WIESCHE, Erik, 22453 Hamburg (DE)
(86) Internationale Anmeldenummer: PCT/EP2019/060727
(87) Internationale Veröffentlichungsnummer: WO 2019/219350

(56) Entgegenhaltungen:
- EP-A1- 1 629 775
- DE-A1- 102016 212 202
- DE-A1- 102016 225 674
- JACHOWICZ J: "Hair damage and attempts to its repair", JOURNAL OF THE SOCIETY COSMETIC CHEMISTS, SOCIETY OF COSMETIC CHEMISTS, US, vol. 38, no. 4, 1987, pages 263 - 286, XP009514043, ISSN: 0037-9832
- KREPLAK L ET AL: "New Aspects of the [alpha]-Helix to [beta]-Sheet Transition in Stretched Hard [alpha]-Keratin Fibers", BIOPHYSICAL JOURNAL, vol. 87, no. 1, 2004, AMSTERDAM, NL, pages 640 - 647, XP055600931, ISSN: 0006-3495, DOI: 10.1529/biophysj.103.036749
- ZOCCOLA M ET AL: "Characterisation of keratin biomass from butchery and wool industry wastes", JOURNAL OF MOLECULAR STRUCTURE, ELSEVIER, AMSTERDAM, NL, vol. 938, no. 1-3, 16 December 2009 (2009-12-16), pages 35 - 40, XP026771992, ISSN: 0022-2860, [retrieved on 20090902], DOI: 10.1016/J.MOLSTRUC.2009.08.036
- ANTUNES E ET AL: "Insights on the mechanical behavior of keratin fibrils", INTERNATIONAL JOURNAL OF BIOLOGICAL MACROMOLECULES, ELSEVIER BV, NL, vol. 89, 6 May 2016 (2016-05-06), pages 477 - 483, XP029601617, ISSN: 0141-8130, DOI: 10.1016/J.IJBIOMAC.2016.05.018

## Beschreibung

Die Erfindung betrifft eine Anordnung zum Bestimmen eines Dehnungsgrades von Haar, ein Verfahren zum Bestimmen des Dehnungsgrades von Haar, und ein Computerprogrammprodukt, welches ausgeführt ist, die Schritte eines solchen Verfahrens auszuführen, wenn es auf einer entsprechenden Anordnung ausgeführt wird.

Bei einer Behandlung von Haar mit kosmetischen Produkten kann eine Wirkung des Produkts, z.B. eine Intensität einer Färbung, stark von einer Schädigung des Haars abhängen.

Haar kann durch natürliche oder künstlich herbeigeführte Vorgänge geschädigt werden. Relevant für die Wirkung eines Produkts können sowohl äußere oder auch innere Schäden von Haar sein. Allgemein kann Haar auf Grund von chemischen oder physikalischen Prozessen oder auf Grund von mechanischen Einwirkungen geschädigt werden.

Die natürlichen Vorgänge können beispielsweise eine kombinierte (z.B. gleichzeitige) Einwirkung von UV-Licht und Sauerstoff (O₂) auf das Haar aufweisen. Die künstlich herbeigeführten Vorgänge können dabei beispielsweise ein Anwenden von Haarfärbemitteln (auch als Colorationen bezeichnet), ein Blondieren, und/oder ein Erzeugen einer Dauerwelle umfassen.

Dabei kann neben erwünschten kosmetischen Effekten, wie z.B. einer Aufhellung des Haars, auch eine starke Schädigung des Haars auftreten, beispielsweise bei einer Verwendung von Oxidationsmitteln.

Der hieraus resultierende Schädigungstyp wird als oxidative Schädigung bezeichnet und wird durch die Anwendung von Colorationen, Blondierungen, Dauerwellen und auch durch Umwelteinflüsse (UV + O₂) hervorgerufen. Diese Schädigung wird ursächlich durch die Oxidation der im Haar sehr häufig vorkommenden Aminosäuren Cystin und Cystein zur Cysteinsäure hervorgerufen.

Cystin kann im Haar intermolekulare Disulfidbrücken (auch als S-S-Brücken bezeichnet) ausbilden, so dass das Cystin äußerst wichtig ist für die mechanische Stabilität des Haars.

Die Oxidation dieser Brücken zu Cysteinsäure kann die mechanische Stabilität des Haars zerstören und bei mehrfachen Anwendungen sogar zu einem vollständigen Haarbruch führen. Allerdings können bereits vorher makroskopisch wahrnehmbare, z.B. fühlbare, Eigenschaften des Haars, beispielsweise eine Oberflächenrauigkeit, negativ beeinflusst werden. Auch Ergebnisse kosmetischer Behandlungen, insbesondere schädigender Verfahren, können bereits in einem frühen Schädigungsstadium massiv verändert sein im Vergleich zu einem Ergebnis bei ungeschädigtem Haar.

Neben dieser oxidativen Schädigung ist auch eine reduktive Schädigung des Haares möglich. Diese tritt bei kosmetischen Verfahren auf, die Reduktionsmittel nutzen. Dies sind z.B. Dauerwellen oder Straightener, die Reduktionsmittel wie z. B. Thioglykolsäure oder Sulfit enthalten. Diese Inhaltsstoffe dienen der Öffnung der Disulfidbrücken des Cysteins zwecks Umformung der Haare. Dabei werden folgende Schwefel-Spezies ausgebildet: R-S-H (Thiole), R-S-SO₃-, (Bunte Salze, nach Sulfit-Behandlung), R-S-S-CH2COO⁻ (Disulfide mit Thiglykolat-Einheiten, nach Thioglykolat-Behandlung).

Weiterhin ist als meist mechanische Schädigung ein Dehnungsgrad von Haar ein relevanter Parameter, insbesondere bei irreversibler Überdehnung (d.h. eine Überdehnung, welche 10% der ungedehnten Länge übersteigt, gemessen beispielsweise bei einer relativen Feuchte einer Haarprobe von 45%), um die Wirkung von Pflege- und/oder Behandlungsmitteln auf das Haar im Voraus abschätzen oder gar geeignet auswählen zu können.

In einem akademischen und industriellen Bereich können einem Forscher oder Entwickler eine Vielzahl von physikalischen, chemisch-analytischen und/oder biophysikalischen Verfahren zur Verfügung stehen, um eine Ermittlung des inneren und äußeren Schädigungsgrads von Haar durchzuführen. Herkömmlich werden hierbei chromatographische Verfahren verwendet, wie beispielsweise Hochleistungsflüssigkeitschromatographie (HPLC) nach einem aufwändigen sauren hydrolytischen Aufschluss der Haarprobe. Solche Verfahren sind in der Regel apparativ sehr aufwändig und für Endanwender oder Verbraucher meist nicht nutzbar, weil schlicht der Zugang dazu fehlt.

Schädigende kosmetische Behandlungen, beispielsweise Haarcolorationen, Hitzeanwendungen, Dauerwellen oder oxidative Prozeduren wie z.B. Blondieren, und viele andere mehr, werden typischerweise im privaten Bereich oder im Bereich kommerzieller Dienstleistungen am Endverbraucher ausgeführt. Obwohl eine Durchführung eines weiteren schädigenden Verfahrens an vorgeschädigtem Haar zu katastrophalen Ergebnissen bis hin zu einem vollständigen Haarbruch führen kann, bestand in diesem Rahmen bisher keine Möglichkeit, den Dehnungsgrad des Haars auf einfache Weise zu bestimmen.

Weiterhin gibt es viele verschiedene Haarbehandlungsprodukte am Markt, welche unterschiedliche Haareigenschaften oder-parameter, wie beispielsweise den Glanz, verbessern sollen. In vielen Fällen kennt der Anwender solcher Produkte allerdings den Schaden des Haares nicht. Dies kann dazu führen, dass der Anwender zu in seinem speziellen Fall weniger geeigneten Produkten greift und nach ihrer Anwendung mit deren Wirksamkeit unzufrieden ist.

Dokument DE102016212202 A1 beschreibt ein Verfahren zur Bestimmung des Schädigungsgrades von Haaren durch Analyse von Nahinfrarotspektren. Dokument EP1629775 A1 beschreibt ein Verfahren zur Bestimmung von Haarschädigung mittels multivariater Komponentenanalyse eines Nahinfrarotabsorptionsspektrums von Haar.

Deshalb kann die Ermittlung eines Dehnungsgrades des Haars von großer Bedeutung sein.

Daneben kann es vorteilhaft sein, ein auf individuelle Bedürfnisse abgestimmtes Produkt anzubieten.

Es kann als Aufgabe der vorliegenden Erfindung betrachtet werden, das Bestimmen eines Dehnungsgrades von Haar zu vereinfachen, insbesondere was den apparativen und zeitlichen Aufwand angeht.

Diese Aufgabe wird gelöst mit den Merkmalen der unabhängigen Ansprüche. Weiterbildungen der Erfindung ergeben sich aus den abhängigen Ansprüchen und aus der folgenden Beschreibung.

Der Erfindung liegen unter anderem die folgenden Erkenntnisse zu Grunde: Es wurde erkannt, dass die Überdehnung von Haaren (der Dehnungsgrad) ein relevanter kosmetischer Parameter ist und dass dieser Dehnungsgrad mit sehr geringem Aufwand mit Hilfe von Nahinfrarotsensoren festgestellt werden kann. Dies ermöglich ein nicht-mechanisches und berührungsloses Bestimmen des Dehnungsgrades von Haar. Insbesondere kann der Dehnungsgrad in einem Wellenlängenbereich des nahen Infrarot (liegt allgemein in einem Wellenlängenbereich von 780 bis 3000 nm) bestimmt werden, was die Verwendung eines apparativ wenig aufwändigen Nahinfrarotsensors ermöglicht.

Menschliches Haar besteht im Wesentlichen aus den dominierenden Elementen der Cuticula-Zellen und Cortex-Zellen. In den Cortex-Zellen lassen sich neben amorphen Bestandteilen komplex aufgebaute fibrilläre Strukturen finden, die dem Haar seine mechanische Stabilität verleihen. Grundbaustein dieser komplexen Faserhierarchie sind als Sekundärstruktur sogenannte α-Helices. Neben den α-Helices sind sogenannte β-Faltstrukturen die verbreitetste Form von Sekundärstrukturen. Bezogen auf die Länge pro Aminosäure-Einheit sind α-Helices im Vergleich zu den β-Faltstrukturen wesentlich kompakter gebaut. Pro Aminosäure beträgt die Länge der α-Helices etwa 0,15 nm, während in der Faltblattstruktur pro Aminosäure 0,35 nm zur Verfügung stehen, was einem Faktor von deutlich über 2 entspricht. Beim mechanischen Dehnen von Haaren ist eine Umwandlung von α-Helices in β-Faltblattstrukturen zu beobachten. Dies stellt einen Umwandlungsprozess bestehender Strukturelemente dar und verändert die Struktur von Haar, was in einem Absorptionsspektrum einer Haarprobe im Infrarotbereich erkannt werden kann.

Gemäß einem Aspekt ist eine Anordnung zum Bestimmen eines Dehnungsgrades von Haar mit den Merkmalen des unabhängigen Anspruchs 1 angegeben. Die Anordnung weist eine Erfassungseinheit zum Erfassen von Haarmerkmalen und eine Auswerteeinheit zum Auswerten der erfassten Haarmerkmale und zum Ermitteln des Dehnungsgrades von Haar basierend auf den erfassten Haarmerkmalen auf. Die Erfassungseinheit enthält einen Nahinfrarotsensor, NIRS und ist ausgeführt, eine Haarprobe mit elektromagnetischen Wellen im Infrarotbereich zu bestrahlen und einen Absorptionsgrad der Haarprobe in einem Wellenlängenbereich von 800 bis 2500 nm zu erfassen. Die Erfassungseinheit ist weiter ausgeführt, ein Absorptionsspektrum der Haarprobe in dem Wellenlängenbereich von 800 bis 2500 nm zu generieren und der Auswerteeinheit bereitzustellen. Die Auswerteeinheit ist ausgeführt, das Absorptionsspektrum mit einem Kalibriermodell abzugleichen und einen Dehnungsgrad der Haarprobe basierend auf dem Absorptionsspektrum und dem Kalibriermodell zu ermitteln.

Es wurde erkannt, dass sich ein Absorptionsspektrum in Abhängigkeit der Struktur von Haar ändert und dass für das Ermitteln eines Dehnungsgrads oder einer mechanischen Überdehnung von Haar insbesondere das Absorptionsspektrum in einem Wellenlängenbereich von 800 bis 2500 nm (jeweils einschließlich dieser Grenzen) maßgeblich ist. Die Schwingungsfrequenz und somit auch das Absorptionsspektrum von überdehntem Haar ändert sich auf Grund seiner Struktur. Eine Überdehnung wandelt α-Helices in β-Faltblattstrukturen um, womit sich das Absorptionsspektrum ändert.

Weiterhin wurde erkannt, dass mit einem Nahinfrarotsensor in dem Wellenlängenbereich von 800 nm bis 2500 nm ein Absorptionsspektrum einer Haarprobe erfasst und/oder generiert werden kann, und dass das Absorptionsspektrum in diesem Wellenlängenbereich Hinweise auf den Dehnungsgrad von Haar enthält. Insbesondere kann der Dehnungsgrad von Haar damit in einem Wellenlängenbereich unterhalb von 3000 nm bestimmt werden. Dies ermöglicht es, apparativ wenig aufwändige Nahinfrarotsensoren zu verwenden. Es ist nicht nötig, auf apparativ viel aufwändigere Sensoren für den mittleren oder fernen Infrarotbereich zurückzugreifen.

Als nahes Infrarot (NIR) im Sinne dieser Beschreibung werden elektromagnetische Wellen mit einer Wellenlänge zwischen 780 nm und 3000 nm (jeweils inklusive) bezeichnet. Wie an anderer Stelle in diesem Dokument beschrieben, werden aus diesem Wellenlängenbereich bestimmte kleinere Wellenlängenbereiche ausgewählt, um ein Absorptionsspektrum einer Haarprobe zu erstellen und um aus diesem Spektrum auf die innere Struktur der Haarprobe zu schließen.

In dem Wellenlängenbereich zwischen 800 und 2500 nm können Oberschwingungen der α-Helices und β-Faltblattstrukturen erfasst werden, welche ein Indikator für den Dehnungsgrad von Haar sind. Insbesondere kann der Wellenlängenbereich zwischen 2000 und 2500 nm, weiter insbesondere zwischen 2000 und 2350 nm herangezogen werden, um das Absorptionsspektrum zu ermitteln und für das Bestimmen des Dehnungsgrades heranzuziehen. Der betrachtete Wellenlängenbereich kann als relevanter Wellenlängenbereich bezeichnet werden.

Oberschwingungen der α-Helices können insbesondere auftreten bei zumindest einer der Wellenlängen aus einer ersten Gruppe von 2012 nm, 2056 nm, 2168 nm, 2183 nm, 2206 nm, 2293 nm, 2345 nm. Oberschwingungen der β-Faltblattstrukturen können insbesondere auftreten bei zumindest einer der Wellenlängen aus einer zweiten Gruppe von 2055 nm, 2166 nm, 2208 nm, 2297 nm, 2343 nm. Der jeweilige Absorptionsgrad der Haarprobe bei zumindest einer Wellenlänge aus jeder der beiden obigen Gruppen kann anzeigen, wie hoch der Anteil von α-Helices und β-Faltblattstrukturen in der Haarprobe ist.

Es kann natürlich auch das gesamte Absorptionsspektrum über mehr als einen Wellenlängenwert aus jeder der beiden obigen Gruppen herangezogen werden, um den Dehnungsgrad zu bestimmen. Der herangezogene Bereich des Absorptionsspektrums entspricht dem oben genannten relevanten Wellenlängenbereich.

In einem Kalibriermodell können die Absorptionsspektren von Haarproben in dem relevanten Wellenlängenbereich mit verschiedenen Dehnungsgraden hinterlegt sein. Jeder Haarprobe und jedem Absorptionsspektrum ist ein Dehnungsgrad zugeordnet und der Dehnungsgrad desjenigen Absorptionsspektrums aus dem Kalibriermodell, welches dem Absorptionsspektrum der untersuchten Haarprobe qualitativ und/oder quantitativ am ähnlichsten ist, wird der untersuchten Haarprobe zugewiesen.

Insbesondere wird das erfasste Absorptionsspektrum der Haarprobe in dem relevanten Wellenlängenbereich mit dem relevanten Wellenlängenbereich der Absorptionsspektren des Kalibriermodells abgeglichen.

Die Dehnung kann als Verhältnis der Länge eines gedehnten Haars zu einem ungedehnten Haar angegeben werden.

Dabei ist die Dehnung eine dimensionslose Angabe. Wird Haar lediglich innerhalb des vollständig reversiblen Bereichs (typischerweise bis 3% bis 5% der Ausgangslänge, d.h. ungedehnten Länge) gedehnt, nimmt es nach der Dehnung stets seine Ausgangslänge ein. Lediglich wenn das Haar über den reversiblen Bereich hinaus (ab ca. 10% bis 15% Dehnung setzt die teilweise irreversible Umwandlung von α-Helices in β-Faltblattstrukturen ein, ab ca. 25% beginnen die Haare zu reißen) gedehnt wird, wird es nicht mehr vollständig auf die ungedehnte Länge zurückkehren, weil dabei eine irreversible Umwandlung von α-Helices in β-Faltblattstrukturen erfolgt. Verschieden starke Dehnungen können als Dehnungsgrade angegeben werden. Die Skala zwischen nicht gedehnt bis hin zum Reißen eines Haars kann in zwei oder mehrere Dehnungsgrade unterteilt werden.

Als Dehnungsgrad können beispielsweise die folgenden Grade genutzt werden: nicht gedehnt, leicht gedehnt, stark gedehnt, mehrfach stark gedehnt. Um die Dehnungsgrade in dem Kalibriermodell zu bestimmen, können andere chemische oder physikalische sowie biophysikalische Verfahren genutzt werden.

Die Auswerteeinheit kann ein Prozessor oder ein Computer sein, welcher Signalverarbeitungsalgorithmen implementiert und ausführt, um basierend auf den von dem NIR-Sensor erfassten Signalen das Absorptionsspektrum der Haarprobe zu ermitteln.

Eine Haarprobe kann ein einzelnes Haar sein, enthält aber bevorzugt eine Vielzahl von einzelnen Haaren, um die Intensität des von der Haarprobe abgegebenen Lichts als Umkehrung des von der Haarprobe absorbierten Lichts (oder die Menge des abgegebenen Lichts in Bezug zu dem abgestrahlten Licht) im Vergleich zu einem einzelnen Haar zu erhöhen. Beispielsweise kann Haar zerstörungsfrei untersucht werden, indem die Erfassungseinheit auf das Kopfhaar einer Person gerichtet wird. In anderen Worten kann das Haar untersucht werden, ohne es abschneiden zu müssen.

Das Absorptionsspektrum kann aus dem von der Haarprobe abgegebenen Licht ermittelt werden. Unter einem Spektrum von Licht wird vorliegend die Absorption des Lichts über der Wellenlänge verstanden. Bei dem Spektrum kann es sich um eine kontinuierliche Angabe der Absorption des Lichts in einem Wellenlängenbereich von einem unteren Wert zu einem oberen Wert handeln. Diese kontinuierliche Angabe beschreibt den Absorptionsgrad des Lichts über der Wellenlänge und hat einen für die Struktur der einzelnen Haare der Haarprobe charakteristischen Verlauf.

Der Nahinfrarotsensor ermöglicht es, Methoden der Nahinfrarotspektroskopie zu nutzen. Diese Methoden erlauben eine direkte, zerstörungsfreie Prüfung der Struktur von Haar ohne eine aufwändige Probenvorbereitung und ohne das Haar durch die Analyse in seiner Struktur zu verändern oder zu zerstören.

Dies kann ein schnelleres Erzielen von Ergebnissen ermöglichen. Darüber hinaus kann es möglich sein, die Haare nach der Messung weiteren Behandlungen zu unterziehen, so dass an einer Haarprobe (beispielsweise in Form einer Haarsträhne) Mehrfachanwendungen oderuntersuchungen durchgeführt werden können.

Zudem kann es möglich sein, relativ leicht Messungen an verschiedenen Haarpositionen durchzuführen (z.B. kopfhautnahes und kopfhautentferntes Haar), beispielsweise auch direkt am Kopf, ohne die Haarprobe entnehmen zu müssen.

Die Nahinfrarotspektroskopie kann beispielsweise dann angewendet werden, wenn eine Probenaufbereitung schwierig oder unmöglich ist und in kurzer Zeit eine Vielzahl von Proben zu analysieren ist, z.B. in einer pharmazeutischen Produktion zur Chargenfreigabe oder für Messungen an Haut oder an anderen biologischen Objekten.

Die Nahinfrarotspektroskopie basiert auf einer Messung spektraler Absorption durch Schwingungsanregungen chemischer Bindungen oder Strukturen der Haarprobe. Abhängig davon, um welche Art von Bindung oder Struktur es sich handelt, kann eine Absorption bei unterschiedlichen Wellenlängen stattfinden. In der Regel kann die Absorption dem Lambert-Beerschen Gesetz folgen und damit proportional zu einer Konzentration eines für die Absorption verantwortlichen Bestandteils sein. Überlagernde Absorptionen sind additiv, so dass eine Quantifizierung auf Basis der Absorptionsspektren möglich ist.

Allerdings kann es sich bei diesen Absorptionen um Obertöne (z.B. im NIR-Bereich) oder Kombinationsschwingungen handeln. Eine Zuordnung einzelner Absorptionswellenlängen kann dadurch erschwert sein.

Aus diesem Grunde können zur Auswertung der Spektren und insbesondere zur quantitativen Evaluierung chemometrische Verfahren eingesetzt werden, um das Kalibriermodell als Ausgangspunkt für das Zuordnen einer Schadenskategorie zu nutzen.

Die NIR Spektroskopie kann auch deshalb geeignet sein zur Messung an Haaren, weil mit der Nahinfrarotstrahlung nicht nur die Oberfläche der Haare analysiert wird, sondern wegen der kleinen Absorptionsquerschnitte für die nahinfrarote Strahlung (z.B. verglichen mit Licht im sichtbaren Wellenlängenbereich) die Haare zumindest teilweise durchdrungen werden können und auch die innere Beschaffenheit von Haar Berücksichtigung findet, was insbesondere für das Ermitteln eines Dehnungsgrades relevant sein kann.

Eine Auswertung der Spektren kann mittels eines Rechenmodells erfolgen, welches in einer Kalibrierphase und Validierphase anhand eines genügend großen Kollektivs von Vergleichsspektren entwickelt werden kann. Dazu kann es erforderlich sein, dass für alle Spektren Referenzwerte der zu kalibrierenden Kenngrößen vorliegen.

Das Rechenmodell kann ein künstliches System sein, das beispielsweise aus den KalibrierHaarproben lernt und diese nach Beendigung der Lernphase verallgemeinern kann. Das heißt, es werden nicht einfach die Beispiele auswendig gelernt, sondern es werden Muster und Gesetzmäßigkeiten in den Lerndaten erkannt. Hierzu können unterschiedliche Ansätze verfolgt werden. Beispielsweise kann ein überwachtes Lernen, ein teilüberwachtes Lernen, ein unüberwachtes Lernen, ein bestärktes Lernen und/oder ein aktives Lernen eingesetzt werden, insbesondere in Verbindung mit sogenannten deep learning-Verfahren. Ein überwachtes Lernen kann beispielsweise mittels eines künstlichen neuronalen Netzes (etwa einem rekurrenten neuronalen Netz) oder mittels einer Support Vector Machine erfolgen. Auch ein unüberwachtes Lernen kann beispielsweise mittels eines künstlichen neuronalen Netzes (beispielsweis eines Autoencoders), Entscheidungsbäumen oder unter Anwendung von Ensemblemethoden (beispielsweise sog. decision tree ensembles) erfolgen.

Spektren können grundsätzlich in Transmission oder Reflektion gemessen werden. Bei der Reflektion werden die von einer Probe reflektierten elektromagnetischen Wellen erfasst und bei der Transmission werden die die Probe passierenden/transmittierenden elektromagnetischen Wellen erfasst. Die reflektierten oder passierenden/transmittierenden elektromagnetischen Wellen werden im Sinne dieser Beschreibung als abgegebene elektromagnetische Wellen oder abgegebenes Licht bezeichnet.

Das Absorptionsspektrum wird generiert, indem die von der Erfassungseinheit erfassten elektromagnetischen Wellen (entspricht dem von der Haarprobe abgegebenen Licht) mit den anfänglich emittierten elektromagnetischen Wellen verglichen oder hiervon subtrahiert werden. Die Intensität über der Wellenlänge stellt das Spektrum dar. Die Differenz der Intensität über der Wellenlänge zwischen dem emittierten NIR-Licht und dem erfassten NIR-Licht stellt das Absorptionsspektrum dar.

Gemäß einer Ausführungsform ist die Auswerteeinheit ausgeführt, einen Verlauf des Absorptionsspektrums der Haarprobe mit einer Mehrzahl von Absorptionsspektren aus dem Kalibriermodell abzugleichen.

Das Kalibriermodell kann in einem lokalen Speicher der Auswerteeinheit vorliegen. Alternativ kann die Auswerteeinheit auf eine externe Datenspeichereinheit zugreifen und die Daten des Kalibiermodells für eine temporäre Nutzung abrufen. Damit kann das Kalibriermodell an einer zentralen Stelle vorgehalten und bei Bedarf geändert werden. So steht der (dezentralen) Auswerteeinheit stets ein aktueller Datensatz des Kalibriermodells zur Verfügung.

Gemäß einer weiteren Ausführungsform ist die Erfassungseinheit ausgeführt, einen Feuchtigkeitsgrad der Haarprobe zu erfassen oder zu erhalten und den Feuchtigkeitsgrad der Auswerteeinheit bereitzustellen, und die Auswerteeinheit ist ausgeführt, den Feuchtigkeitsgrad der Haarprobe beim Abgleich des generierten Absorptionsspektrums mit dem Kalibriermodell zu berücksichtigen.

Der Feuchtigkeitsgrad der Haarprobe kann beispielsweise durch einen Nutzer eingegeben werden oder von einem Sensor erfasst werden. Dieser Sensor kann Teil der Erfassungseinheit sein.

Es wurde gefunden, dass gedehntes oder überdehntes Haar seinen Feuchtigkeitsgehalt oder Wassergehalt ändert. Dadurch ändert sich auch das Absorptionsspektrum des Haars. Ist das Haar einer äußeren Feuchtigkeitsquelle ausgesetzt, beispielsweise hoher Luftfeuchtigkeit oder Regen, so kann dies Einfluss auf das Absorptionsspektrum in dem genannten Wellenlängenbereich haben und auch auf den zugeordneten Dehnungsgrad. Um den Einfluss von Feuchtigkeit auf das Messergebnis zu eliminieren, wird der Feuchtigkeitsgrad der Haarprobe berücksichtigt.

Um den Feuchtigkeitsgrad anzugeben, kann beispielsweise schon eine Angabe über die Luftfeuchtigkeit ausreichen. Die Luftfeuchtigkeit kann beispielsweise als relative Luftfeuchtigkeit angegeben werden, welche in Gewichts-% angegeben wird und das Gewichtsverhältnis eines momentanen Wasserdampfgehaltes zu einem bei der aktuellen Temperatur maximal möglichen Wasserdampfgehalt in der Luft angibt. Auch kann der Feuchtigkeitsgrad als Gewichtsanteil von Wasser an dem Gesamtgewicht der nassen Haarprobe (Eigengewicht des Haares + Gewicht des Wassers) angegeben werden. Vereinfacht kann dann der Feuchtigkeitsgrad beispielsweise als eine von mehreren Stufen angegeben werden: leicht feucht, feucht, nass, sehr nass.

Gemäß einer weiteren Ausführungsform ist die Auswerteeinheit ausgeführt, der Haarprobe basierend auf dem Abgleich des generierten Absorptionsspektrums mit dem Kalibriermodell eine Schadenskategorie zuzuordnen.

Die bekannten und verfügbaren Absorptionsspektren von Haarproben können Teil des Kalibriermodells sein. Das Kalibriermodell kann beispielsweise basierend auf einer Mehrzahl von Kalibrierhaarproben erstellt werden, wobei für jede einzelne Kalibrierhaarprobe ein Kalibrierspektrum erfasst wird (wie auch oben für das Erzeugen des Spektrums der zu untersuchenden Haarprobe beschrieben) und die Kalibrierhaarprobe mit anderen unabhängigen analytischen Verfahren auf Schäden untersucht, und der damit ermittelte Schaden der Kalibrierhaarprobe zugeordnet wird.

Durch einen Abgleich der Spektren der Kalibrierhaarproben mit dem Spektrum der zu untersuchenden Haarprobe kann der Dehnungsgrad für letztere ermittelt werden. Bei diesem Abgleich kann die Form des Spektrums und/oder dessen Intensität berücksichtigt werden. Die Berücksichtigung der Form kann auch dann einen Treffer beim Abgleich liefern, wenn die Struktur der Haare der untersuchten Haarprobe und der Kalibrierhaarproben nicht übereinstimmen.

Das Kalibriermodell kann in einem lokalen Speicher der Anordnung oder in einer externen Datenspeichereinheit gespeichert sein. Die Erfassungseinheit nimmt zumindest einen Teil eines Spektrums von NIR-Licht, welches mit der Haarprobe gewechselwirkt hat, auf und gleicht dieses Spektrum mit einer Mehrzahl von Kalibrierhaarproben ab. Für diesen Abgleich wird beispielsweise ein Prozessor der Auswerteeinheit verwendet und es wird zumindest ein Teil des Spektrums der untersuchten Haarprobe mit dem spektroskopischen Kalibriermodell zum Ermitteln des Dehnungsgrades des Haars verglichen.

Gemäß einer weiteren Ausführungsform ist der Nahinfrarotsensor ein Spektrometer, wobei das Spektrometer ausgeführt ist, basierend auf dem erfassten Licht ein Signalmuster zu erstellen, welches charakteristisch für die erfassten Haarmerkmale (können allgemein auch als Haareigenschaften bezeichnet werden) ist.

Dies bedeutet, dass nicht zwingend absolute Werte für Merkmale oder Eigenschaften des Haars bestimmt werden müssen. Vielmehr kann es ausreichen, die erhaltenen Signalmuster des Spektrometers zu nutzen, um eine Produktempfehlung und/oder einen Anwendungshinweis für die Behandlung und/oder Pflege des Haars zu ermitteln. Das Signalmuster kann Teil einer jeden Kalibrierprobe des Kalibriermodells sein. Es kann unabhängig hiervon ermitteln werden, welche Produkte und/oder Anwendungshinweise für Haarproben mit einem bestimmten Signalmuster sinnvoll sind. Den so ermittelten Produkten und/oder Anwendungshinweisen kann dann das entsprechende Signalmuster zugeordnet werden, um auszudrücken, dass sich diese Produkte und/oder Anwendungshinweise für Haar mit diesem Signalmuster eignen und um eine bestimmte Wirkung zu erzielen. Dies bedeutet, dass das Auffinden einer geeigneten Produktempfehlung vereinfacht wird, weil nur die ermittelten Signalmuster der untersuchten Haarprobe mit Signalmustern, die den Produkten und/oder Anwendungs- und Behandlungshinweisen zugeordnet sind, verglichen werden müssen.

Gemäß einer weiteren Ausführungsform weist die Anordnung weiterhin ein Gehäuse und einen Energiespeicher auf, wobei die Auswerteeinheit in dem Gehäuse untergebracht und die Erfassungseinheit mit dem Gehäuse gekoppelt ist, und wobei der Energiespeicher in dem Gehäuse angeordnet ist, um die Auswerteeinheit mit Energie zu versorgen, und um zumindest zeitweise einen autarken Betrieb der Auswerteeinheit ohne Anschluss an eine externe Energiequelle zu ermöglichen.

Dies bedeutet, dass die Auswerteeinheit autark und ohne Zufuhr von Energie von außen wie vorgesehen betrieben werden können. Der Energiespeicher ist bevorzugt ein wiederaufladbarer Energiespeicher. In einem Ausführungsbeispiel ist die Nutzerschnittstelle auch in dem Gehäuse angeordnet und kann von der Energiequelle mit Energie versorgt werden, um ebenfalls autark betrieben zu werden. In einem weiteren Ausführungsbeispiel kann der Energiespeicher auch die Erfassungseinheit mit Energie versorgen.

Das Gehäuse und die Auswerteeinheit können Bestandteil eines persönlichen Geräts eines Nutzers sein. Das persönliche Gerät kann ein portabler Computer in Form eines Smartphones, eines Tablets oder eines anderen Computers sein (diese Einheiten können allgemein als Recheneinheit oder portable Recheneinheit bezeichnet werden). Die Erfassungseinheit kann an die Recheneinheit angeschlossen oder darin integriert sein und wie hierin beschrieben zum Ermitteln des Dehnungsgrades von Haaren verwendet werden. In einem Computerprogrammprodukt (z.B. Software oder Anwendung für das persönliche/portable Gerät) werden die erfassten Merkmale des Haars dann in Form von Werten, willkürlichen Einheiten angezeigt oder akustisch ausgegeben. Aus den Parametern können dann (a) Produktempfehlungen für individuell passende Behandlungsprodukte und individuelle Behandlungstipps abgeleitet und/oder (b) der Behandlungserfolg bei einer kosmetischen Behandlung, die das Ziel hat, die gemessen Parameter positiv zu beeinflussen, ermittelt und/oder angezeigt werden.

Die Erfassungseinheit kann eine Schnittstelle (auch: Datenübertragungsverbindung) aufweisen, über welche eine Verbindung mit der Recheneinheit hergestellt wird. Die Recheneinheit kann eine erste Schnittstelle und eine zweite Schnittstelle aufweisen. Die erste Schnittstelle kann als Gegenstück zu der Schnittstelle der Erfassungseinheit ausgeführt sein, also um die Erfassungseinheit an die Recheneinheit anzubinden. Die zweite Schnittstelle kann ausgeführt sein, die Recheneinheit mit einem Datennetz zu verbinden. Diese Verbindungen sind ausgeführt, Informationen in mindestens eine Richtung zu übertragen, bevorzugt in beide Richtungen. Die Verbindung zwischen Erfassungseinheit und Recheneinheit einerseits und die Verbindung zwischen Recheneinheit und einem Zugangspunkt des Datennetzes können drahtgebunden oder drahtlos sein. Drahtgebundene Verbindungen können beispielsweise optische oder elektrische Signale für die Informationsübertragung nutzen. Drahtlose Verbindungen nutzen typischerweise elektromagnetische Wellen für die Signalübertragung, z.B. Funksignale oder auch optische Signale.

Für die Anbindung der Erfassungseinheit an die Recheneinheit können Protokolle verwendet werden, die gemäß den Grundsätzen vermaschter Netze (mesh network) arbeiten. Beispielsweise kann für die Datenübertragung und für die Anbindung der Erfassungseinheit an die Recheneinheit das Thread-Protokoll verwendet werden, welches auf IPv6 aufsetzt. Das Thread-Protokoll wird insbesondere eingesetzt, um automatisierte oder teilautomatisierte Geräte miteinander zu verbinden, in diesem Fall beispielsweise die Erfassungseinheit mit der Recheneinheit.

Die Erfassungseinheit kann in einem Beispiel auf die Recheneinheit strukturell aufgesteckt werden, oder umgekehrt die Recheneinheit auf die Erfassungseinheit. Dies bedeutet, dass die Erfassungseinheit mechanisch an der Recheneinheit oder einem Gehäuse der Recheneinheit befestigt wird. Beispielsweise kann dies durch eine werkzeuglose Montage über eine reversible Verbindung erfolgen. In der aufgesteckten Position kann die Erfassungseinheit mit einer lösbaren kraftschlüssigen oder formschlüssigen Verbindung relativ zu der Recheneinheit gehalten werden. Die Schnittstellen zwischen Erfassungseinheit und Recheneinheit können so angeordnet sein, dass in der aufgesteckten Position eine elektrische Verbindung zwischen Erfassungseinheit und Recheneinheit automatisch hergestellt oder aufgebaut wird.

Die Recheneinheit kann eine Anwendung (oder ein Programm, im Weiteren auch als Computerprogrammprodukt bezeichnet) ausführen, welche Daten von der Erfassungseinheit erhält oder abfragt. Die erhaltenen oder abgefragten Daten werden in der Anwendung verwertet, um einen oder mehrere Ausgabewerte zu ermitteln. Die Daten werden von der Anwendung gemäß der hierin beschriebenen Ansätze verarbeitet und/oder ausgewertet.

Um die Anwendung auszuführen, können Prozessoren (und einer oder mehrere Speicherbausteine) der Recheneinheit verwendet werden. Die Recheneinheit kann aber auch ausgeführt sein, Rechenschritte für das Ausführen der Anwendung auszulagern. So kann die Anwendung beispielsweise die von der Erfassungseinheit erhaltenen oder abgefragten Daten an eine externe Recheneinheit übertragen. Bevor die Daten an die externe Recheneinheit übertragen werden, können sie einer Vorverarbeitung zugeführt werden.

Die externe Recheneinheit kann räumlich entfernt von der Erfassungseinheit und der portablen Recheneinheit angeordnet sein. Die portable Recheneinheit kann über das Datennetz mit der externen Recheneinheit verbunden sein, d.h. in einer Kommunikationsverbindung stehen. Die externe Recheneinheit kann ein einzelner Computer oder Prozessor oder ein Verbund von Computern oder Prozessoren sein. In einem Rechner- oder Prozessorverbund kann die Rechenlast unter verschiedenen Gesichtspunkten auf die einzelnen Bestandteile des Verbunds verteilt werden. Dieser Rechnerverbund kann neben Rechenleistung auch Speicherkapazitäten für die Benutzer bereitstellen und von den Benutzern freigegebene oder gekennzeichnete Daten vorhalten. Damit kann der in der portablen Recheneinheit benötigte Speicherplatz reduziert werden. Auch wird es dem Benutzer erleichtert, eine portable Recheneinheit auszutauschen, weil lokal keine oder fast keine Daten gespeichert sind. Der Rechnerverbund kann als eine Gruppe von vermascht vernetzten Servern ausgestaltet sein.

Gemäß einer weiteren Ausführungsform ist die Auswerteeinheit ausgeführt, die Merkmale von Behandlungsmitteln zur Behandlung von Haar mit den erfassten Haarmerkmalen abzugleichen und eine Wirkung der Behandlungsmittel auf die Haare unter Berücksichtigung der erfassten Haarmerkmale zu bestimmen.

Dies bedeutet, dass die Behandlungsmittel in Abhängigkeit der ermittelten Haareigenschaften oder erfassten Haarmerkmale ausgesucht und bestimmt werden. Diese Behandlungsmittel können beispielsweise auf der Nutzerschnittstelle angezeigt oder auf andere Weise ausgegeben werden.

Gemäß einer weiteren Ausführungsform ist die Auswerteeinheit ausgeführt, die erfassten Haarmerkmale an eine Datenspeichereinheit zu übertragen und Hinweise zur Behandlung des Haars gemäß den erfassten Haarmerkmalen von der Datenspeichereinheit abzufragen.

Bei diesen Hinweisen kann es sich um allgemeine Hinweise (ohne Bezug zu einem bestimmten Behandlungsmittel) betreffend die Behandlung von Haar handeln, es kann sich aber auch um Hinweise mit Bezug zu einem bestimmten Behandlungsmittel handeln. Die Hinweise können auch Erklärungen beinhalten, welche Verhaltensweisen welche Eigenschaften des Haars wie beeinflussen.

In der Datenspeichereinheit können Informationen aus Studien sowie Informationen aus Literaturquellen und/oder wissenschaftlichen Veröffentlichungen enthalten sein. Die Auswerteeinheit kann ausgeführt sein, einem Nutzer in Abhängigkeit der erfassten Eigenschaften des Haars einen Auszug aus diesen Informationen anzuzeigen, auszugeben oder zumindest darauf hinzuweisen.

Gemäß einer weiteren Ausführungsform weist die Anordnung weiterhin eine Nutzerschnittstelle auf und die Auswerteeinheit ist ausgeführt, die Nutzerschnittstelle anzuweisen, die erhaltenen Hinweise zur Behandlung der Haare auszugeben.

Bei der Nutzerschnittstelle kann es sich beispielsweise um ein Display der portablen Recheneinheit handeln, insbesondere um einen sog. Touchscreen, welcher es ermöglicht, Inhalte optisch anzuzeigen und Benutzereingabe über Berührung entgegenzunehmen.

Gemäß einer weiteren Ausführungsform ist die Auswerteeinheit ausgeführt, Informationen von einem Nutzer abzufragen und diese Informationen beim Abfragen der Datenspeichereinheit zusätzlich zu berücksichtigen, um von der Datenspeichereinheit Merkmale von Behandlungsmitteln zur Behandlung von Haar gemäß der von dem Nutzer abgefragten Informationen zu erhalten.

Die abgefragten Informationen können mittels eines vorgegebenen Fragebogens erfasst werden, bei welchem einer Aussage durch den Nutzer mehr oder weniger Gewicht beigemessen wird oder auch aus einer von mehreren möglichen Antworten ausgewählt wird. Der vorgegebene Fragebogen kann sich insbesondere mit den Lebensgewohnheiten und außergewöhnlichen Belastungen des Nutzers befassen, zum Beispiel Ernährungsgewohnheiten, Dauer und Qualität des Schlafs, Trinkmenge, Art der Getränke, Verwendung von Genussmitteln (beispielsweise Nikotin, Alkohol), beruflichen Aktivitäten und Freizeitaktivitäten (viel Zeit außerhalb von Gebäuden bei jeglicher Witterung, Aufenthalt in den Bergen, Besuch eines Solariums). Es können auch Alter, Geschlecht und Ethnizität der Benutzer abgefragt sowie für das Abfragen des Behandlungsmitteldatenspeichers herangezogen werden. Auch können die abgefragten Informationen sich auf eine gewünschte oder zu erreichende Eigenschaft des Haars beziehen.

Gemäß einer weiteren Ausführungsform weist die Auswerteeinheit einen lokalen Speicher auf, welcher ausgeführt ist, die von der Datenspeichereinheit abgerufenen Daten zu speichern, bevorzugt persistent zu speichern.

Dies bedeutet, dass die Auswerteeinheit zumindest temporär ihre Funktionen ausführen kann, ohne auf eine dauerhafte Verbindung zu der Datenspeichereinheit aber das Datennetz angewiesen zu sein. Die abgerufenen Daten sind in dem lokalen Speicher gespeichert. Die Daten werden in dem lokalen Speicher so abgespeichert, dass sie bei einem Ausschalten oder außer Betrieb setzen der Auswerteeinheit erhalten bleiben (persistente Speicherung). Es ist möglich, dass die Auswerteeinheit lediglich solche Daten aus der Datenspeichereinheit abruft, welche zu einem momentanen Bild oder zu momentanen Eigenschaften des Haars passen. In einer Ausführungsform können auch solche Daten abgerufen und lokal gespeichert werden, welche zu geringfügig geänderten Eigenschaften des Haars ausgehend von dem aktuellen Zustand passen. Es ist also nicht nötig, alle Daten aus der Datenspeichereinheit abzurufen und in dem lokalen Speicher zu speichern. Vielmehr können gezielt diejenigen Daten oder Informationen aus der Datenspeichereinheit in den lokalen Speicher übertragen werden, welche zu dem erfassten Zustand des Haars passen.

Gemäß einer weiteren Ausführungsform ist die Auswerteeinheit ausgeführt, die erfassten Haarmerkmale mit einem Zeitstempel betreffend das Erfassen der Haarmerkmale versehen in dem lokalen Speicher abzuspeichern.

Dies ermöglicht es, Veränderungen des Haars über der Zeit zu beobachten und zu analysieren. Somit können diese Veränderungen auch herangezogen werden, um geeignete nicht-therapeutische Behandlungsmittel und/oder Behandlungshinweise auszugeben. Weiterhin ermöglicht es diese Ausführungsform einem Nutzer, die Veränderungen zu beobachten, um gegebenenfalls das Erreichen von oder das Annähern an selbst gesetzte Ziele feststellen zu können.

Gemäß einer weiteren Ausführungsform ist die Auswerteeinheit ausgeführt, die erfassten Haarmerkmale über einen längeren Zeitraum umfassend zumindest zwei Vorgänge des Erfassens der Haarmerkmale in dem lokalen Speicher abzuspeichern und wahlweise eine Entwicklung der Haarmerkmale über eine vorgebbare Zeitspanne aus dem lokalen Speicher abzurufen und die Ausgabeeinheit anzuweisen, diese Entwicklung anzuzeigen oder auszugeben.

Gemäß einer weiteren Ausführungsform ist die Auswerteeinheit ausgeführt, die erfassten Haarmerkmale an die Datenspeichereinheit zu übertragen.

Die erfassten und übertragenen Haarmerkmale können in der Datenspeichereinheit einer Kennziffer oder einer Kennung eines Nutzers zugewiesen werden, so dass ein Nutzer seine Daten von verschiedenen Geräten einsehen kann. Ebenso hat dieses Vorgehen den Vorteil, dass die Daten eines Nutzers bei Verlust oder Defekt der persönlichen Auswerteeinheit an einer zentralen Stelle gesichert oder hinterlegt sind.

Weiterhin ermöglicht es diese Ausführungsform, Haarmerkmale eines Nutzers über einen längeren Zeitraum zu erfassen und deren Veränderung zu beobachten und gegebenenfalls mit den Empfehlungen für nicht-therapeutische Behandlungsmittel und/oder Behandlungshinweisen zu verknüpfen.

Gemäß einer weiteren Ausführungsform ist die Anordnung ausgeführt, Anweisungen für die Bedienung der Erfassungseinheit visuell auf der Ausgabeeinheit und/oder akustisch über einen Lautsprecher auszugeben. Dies kann insbesondere hilfreich sein, wenn das Haar eines Benutzers erstmalig und umfassend erfasst wird, um einen Überblick über den Zustand des Haars zu erlangen.

Gemäß einer weiteren Ausführungsform ist die Ausgabeeinheit ausgeführt, Informationen über ein Behandlungsmittel, z.B. eine Produktbezeichnung, Informationen betreffend Inhaltsstoffe und/oder Zusammensetzung eines Behandlungsmittels und/oder Anwendungshinweise zur nicht-therapeutischen Behandlung des Haars auszugeben.

Somit wird es einem Nutzer ermöglicht, sich selbst ein Bild über ein Behandlungsmittel in seiner Gesamtheit zu machen. Außerdem können dem Nutzer Anwendungshinweise bezogen auf ein Behandlungsmittel oder unabhängig hiervon gegeben werden. Die Anwendungshinweise können sich auf gewünschtes und/oder ungewünschtes Verhalten beziehen.

Gemäß einer weiteren Ausführungsform ist die Nutzerschnittstelle ausgeführt, nach dem Ausgeben von Merkmalen eines Behandlungsmittels eine Eingabe von einem Nutzer entgegen zu nehmen und basierend auf dieser Eingabe eine Aktion betreffend das angezeigte oder ausgegebene Behandlungsmittel zu veranlassen.

Die Aktion kann sich beispielsweise darauf beziehen, dass dem Nutzer ein Behandlungsmittel zum Kauf angeboten wird und der Nutzer über eine Eingabe den Kauf in die Wege leiten kann. Neben dem Kauf von Behandlungsmitteln können dem Nutzer auch weitergehende Informationen zum käuflichen Erwerb angeboten werden. Diese weitergehenden Informationen können sich auf detailliertere Behandlungs- und Anwendungshinweise beziehen. Das Programm empfängt beispielsweise die Anfrage, dass der Nutzer das Behandlungsmittel erwerben möchte, speichert die Anfrage und/oder übermittelt die Anfrage an ein Handelsunternehmen, welches die Behandlungsmittel vertreibt. Der Nutzer wird durch das Computerprogramm aufgefordert, seine persönlichen Daten (Adresse, Bankinformationen, Versendungspräferenz, etc.) über die Eingabeeinheit einzugeben.

Alternativ kann dem Benutzer ausgegeben werden, wo, beispielsweise in einem Drogeriemarkt, in einem Friseursalon, in einer Apotheke, etc. in seiner Nähe, er das ausgegebene Behandlungsmittel vor Ort erwerben kann.

Immer mehr Kunden wünschen sich ein individuell auf ihre Bedürfnisse abgestimmtes Produkt. Entsprechend kann dem Nutzer ein individuell für ihn hergestelltes Behandlungsprodukt empfohlen und ein Bestellvorgang, beispielsweise durch Aufrufen einer Internetseite eines Herstellers von individuellen Haarbehandlungsmitteln, eingeleitet werden.

Dabei kann es sich um ein speziell für den einen Nutzer hergestelltes Behandlungsmittel oder ein sogenanntes "mass customized" Produkt handeln. Bei einem "mass customized" Produkt kann durch Variation von wenigen, aus Kundensicht jedoch entscheidenden, Merkmalen eines Produkts, eine Individualisierung erreicht werden. Bevorzugt basieren diese "mass customized" Produkte auf dem Konzept der Modularisierung, das heißt, das Produkt kann aus diversen Modulen/Bausteinen individuell zusammengestellt werden.

Zwischen den vielen unterschiedlichen Merkmalen/Inhaltsstoffen eines Produktes bestehen oftmals zahlreiche Abhängigkeiten, die als "Gebote" oder "Verbote" ausgedrückt werden können. Um eine eindeutige Produktdefinition zu erhalten, kann es vorteilhaft sein, dass der Bestellvorgang mit Hilfe eines Produktkonfigurators abläuft. Dieser Konfigurator hilft dem Nutzer bei der Auswahl der Merkmale/Inhaltsstoffe und weist ihn auf die zulässigen/unzulässigen Merkmalskombinationen hin, wobei letztere dann nicht ausgewählt werden können.

Bei Haarbehandlungsmitteln umfassen die relevanten Produktmerkmale insbesondere die chemischen Inhaltsstoffe der Mittel, die physikalischen Eigenschaften der Mittel und die Konfektionsart der Mittel. Mit Hilfe eines Produktkonfigurators kann beispielsweise die Auswahl chemisch und/oder physikalisch inkompatibler Inhaltsstoffe oder die Auswahl für den ermittelten Schädigungsgrad/Dehnungsgrad/etc. ungeeigneter Inhaltsstoffe vermieden werden. Umgekehrt kann die Auswahl für den ermittelten Schädigungsgrad/Dehnungsgrad/etc. geeigneter Inhaltsstoffe durch den Produktkonfigurator vorgegeben oder vorgeschlagen werden.

Ebenso ist es möglich, ein individuelles Haarbehandlungsmittel vor Ort, das heißt zum Beispiel in einem Friseursalon oder an einem Verkaufspunkt von Haarbehandlungsmitteln, wie beispielsweise ein Drogeriemarkt, mittels einer Mischvorrichtung, vorzugsweise einer intelligenten Mischvorrichtung (Smart Mixer), herzustellen.

Gemäß einem weiteren Aspekt ist ein Verfahren zum Bestimmen eines Dehnungsgrades von Haar wie im unabhängigen Anspruch 11 angegeben. Das Verfahren weist die folgenden Schritte auf: Bestrahlen einer Haarprobe mit elektromagnetischen Wellen im Infrarotbereich; Erfassen des von der Haarprobe abgegebenen Lichts; Erfassen eines Absorptionsgrads der Haarprobe in einem Wellenlängenbereich von 800 bis 2500 nm; Generieren eines Absorptionsspektrums der Haarprobe in dem Wellenlängenbereich von 800 bis 2500 nm; Abgleichen des generierten Absorptionsspektrums mit einem Kalibriermodell und Ermitteln des Dehnungsgrades von Haar basierend auf dem Absorptionsspektrum und dem Kalibriermodell.

Dieses Verfahren entspricht der Funktion der oben beschriebenen Anordnung. Die Verfahrensschritte entsprechen dabei den Funktionen der Anordnung und werden an dieser Stelle nicht erneut beschrieben. Jedenfalls wird für Details zu den Verfahrensschritten auf die obige Beschreibung der Anordnung und ihrer Funktion verwiesen. Die übrigen Funktionen der Anordnung können selbstverständlich auch als Verfahrensschritte implementiert werden.

Beim Abgleichen des generierten Absorptionsspektrums mit dem Kalibriermodell kann insbesondere ein Verlauf des Absorptionsspektrums der Haarprobe mit einer Mehrzahl von Absorptionsspektren aus dem Kalibriermodell abgeglichen werden.

Gemäß einer Ausführungsform weist das Verfahren folgenden Schritt auf: Erfassen oder Erhalten eines Feuchtigkeitsgrads der Haarprobe und Heranziehen des Feuchtigkeitsgrads der Haarprobe beim Abgleich des generierten Absorptionsspektrums mit dem Kalibriermodell.

Gemäß einer Ausführungsform weist das Verfahren folgenden Schritt auf: Zuordnen einer Schadenskategorie zu der Haarprobe basierend auf dem Abgleich des generierten Absorptionsspektrums mit dem Kalibriermodell.

Gemäß einem weiteren Aspekt ist ein Verfahren zum Ermitteln eines Behandlungsmittels basierend auf dem bestimmten Dehnungsgrad von Haar angegeben. Das Verfahren weist die folgenden Schritte auf: Heranziehen des bestimmten Dehnungsgrades von Haar; Auswählen eines Behandlungsmittels für Haar basierend auf dem ermittelten Dehnungsgrad und Ausgeben von Informationen über das ausgewählte Behandlungsmittel.

Das Behandlungsmittel wird in Abhängigkeit des ermittelten oder bestimmten Dehnungsgrades und unter Berücksichtigung einer gewünschten Wirkung, beispielsweise gewünschter Eigenschaften der Haare nach der Behandlung, ausgesucht oder ermittelt. Bei der gewünschten Wirkung kann es sich um eine von einem Benutzer vorgegebene Wirkung oder einen gewünschten Zustand des Haars handeln. Hierbei kann es hilfreich sein, dass jedem Behandlungsmittel eine oder mehrere Schadensarten oder Dehnungsgrade zugeordnet werden, bei der eine Anwendung möglich ist, und auch eine Wirkung, welche das Behandlungsmittel bei der entsprechenden Schadensart oder Dehnungsgrad hat. Somit kann über einen einfachen Abgleich der Schadensart oder des Dehnungsgrades und der gewünschten Wirkung das passende Behandlungsmittel ermittelt werden.

Gemäß einem weiteren Aspekt ist ein Computerprogrammprodukt wie im Anspruch 15 angegeben, welches ausgeführt ist, das Verfahren aus Anspruch 11 auszuführen, wenn es auf einer Anordnung aus Anspruch 1 ausgeführt wird.

Das Computerprogrammprodukt ermöglicht die Kontrolle und Nachverfolgung der Ergebnisse durch Darstellung (z.B. graphisch) der Messergebnisse im Verlauf der Zeit. Anhand der erzielten Ergebnisse gibt das Computerprogrammprodukt individuelle Behandlungs- und Produkttipps. Die Qualität der Behandlungs- und Produkttipps kann dadurch verbessert werden, dass der Benutzer zusätzlich Fragen zu seinem Haarzustand, seinen Ernährungsgewohnheiten, seinem generellen Gesundheitszustand und weiteren Verhaltensweisen beantwortet, die das Computerprogrammprodukt entsprechend verarbeiten kann. Dazu werden nicht nur z.B. Literaturdaten zu Grunde gelegt, sondern auch der Behandlungserfolg anderer Nutzer des Computerprogrammprodukts, insbesondere Behandlungserfolge anderer Nutzer, die zumindest einen ähnlichen Haarzustand aufweisen.

Die mittels des Fragebogens erfassten Daten können verwendet werden, um eine Entwicklung des Zustands der Haare des Nutzers unter den gegebenen Umständen, also den von dem Nutzer eingegebenen Daten, zu analysieren. Diese Entwicklung kann mit der Entwicklung anderer Nutzer verglichen werden. Hieraus kann gefolgert werden, ob sich während einer Behandlung mit einem bestimmten Produkt die Entwicklung von Nutzern mit ähnlichen oder gleichen Eingaben im Fragebogen gleicht oder von Nutzern mit anderen Eingaben abweicht.

Die von dem Nutzer eingegebenen Daten können somit für eine globale Analyse verwendet werden, um den Erfolg einer Behandlung und die Wirksamkeit eines Produkts unter verschiedenen Bedingungen überwachen und ggf. Änderungen der Behandlung und/oder des Produkts empfehlen zu können.

Ausführungsbeispiele der Erfindung sind in den Figuren dargestellt und werden im Folgenden näher erläutert. Dabei zeigt:
- Fig. 1: eine schematische Darstellung einer Anordnung zum Ermitteln eines Dehnungsgrades von Haar gemäß einem Ausführungsbeispiel;
- Fig. 2: eine schematische Darstellung eines Datenträgers gemäß einem weiteren Ausführungsbeispiel;
- Fig. 3: eine schematische Darstellung von Schritten eines Verfahrens gemäß einem weiteren Ausführungsbeispiel;
- Fig. 4: eine schematische Darstellung einer Erfassungseinheit für eine Anordnung gemäß einem weiteren Ausführungsbeispiel;
- Fig. 5: eine schematische Darstellung eines Absorptionsspektrums kennzeichnend für den Dehnungsgrad von Haar;
- Fig. 6: eine schematische Darstellung eines Spannungs-Dehnungs-Diagramms von Haar.

In der folgenden ausführlichen Beschreibung wird auf die beigefügten Zeichnungen Bezug genommen, die einen Teil der vorliegenden Anmeldung bilden und in denen zur Veranschaulichung spezifische Ausführungsformen gezeigt sind, in denen die Erfindung ausgeübt werden kann. Es versteht sich, dass andere Ausführungsformen benutzt und strukturelle oder funktionale oder logische Änderungen vorgenommen werden können, ohne von dem Schutzumfang der vorliegenden Erfindung abzuweichen. In dieser Hinsicht wird Richtungsterminologie wie etwa "oben", "unten", "vorne", "hinten", "vorderes", "hinteres", usw. mit Bezug auf die Orientierung der beschriebenen Figur(en) verwendet. Da Komponenten von Ausführungsformen in einer Anzahl verschiedener Orientierungen positioniert werden können, dient die Richtungsterminologie zur Veranschaulichung und ist auf keinerlei Weise einschränkend. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche sowie Äquivalente hiervon definiert.

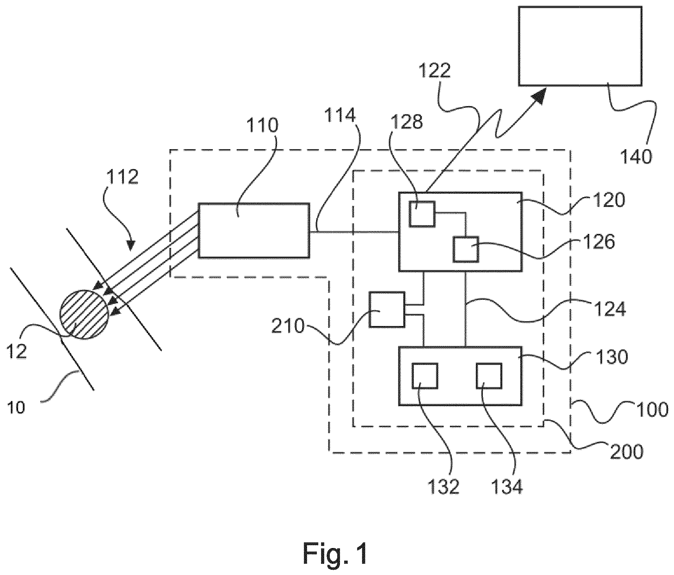

Fig. 1 zeigt eine Anordnung 100 zum Ermitteln eines Dehnungsgrades von Haar. Die Anordnung 100 weist eine Erfassungseinheit 110, eine Auswerteeinheit 120 und eine Nutzerschnittstelle 130 auf. Die Erfassungseinheit 110 ist ausgeführt, Eigenschaften oder Merkmale von Haar zu erfassen. Hierzu emittiert die Erfassungseinheit 110 Infrarotlicht in Richtung eines zu untersuchenden Bereichs 12 des Analyseobjekts 10 (beispielsweise von menschlichem Haar) und erfasst das abgegebene Licht um einen Absorptionsgrad der Haarprobe in einem Wellenlängenbereich von 800 bis 2500 nm zu erfassen. Das von dem zu untersuchenden Bereich 12 abgegebene Licht wird von der Erfassungseinheit 110 aufgenommen und erlaubt Rückschlüsse auf den Dehnungsgrad des Haars, weil die bei gedehntem oder überdehntem Haar geänderte Struktur ein individuelles Absorptionsspektrum in dem erfassten Wellenlängenbereich hat.

Die Erfassungseinheit 110 weist eine geeignete Quelle elektromagnetischer Wellen auf. Diese Quelle ist ein Lichtemitter oder Laseremitter und kann auch als Strahlungsquelle bezeichnet werden und ist an oder in der Erfassungseinheit 110 angeordnet. Die Strahlungsquelle kann so an oder in der Erfassungseinheit 110 angeordnet sein, dass beim Emittieren der elektromagnetischen Wellen 112 die Strahlungsquelle einen vorgegebenen Abstand zu dem zu untersuchenden Bereich 12 einnimmt, insbesondere wenn die Erfassungseinheit 110 auf den zu untersuchenden Bereich gestellt wird. Der Abstand der Strahlungsquelle von dem zu untersuchenden Bereich kann variabel sein und mittels Aktuatoren oder manuell verändert werden.

Die Erfassungseinheit 110 ist über eine Datenübertragungsverbindung 114 mit der Auswerteeinheit 120 verbunden. Die Datenübertragungsverbindung 114 kann eine unidirektionale oder bidirektionale Datenübertragung zwischen der Erfassungseinheit 110 und der Auswerteeinheit 120 ermöglichen. So liefert die Erfassungseinheit 110 Signale betreffend die erfassten Merkmale des Haars an die Auswerteeinheit 120, wohingegen die Auswerteeinheit 120 Steuerbefehle an die Erfassungseinheit 110 liefern kann, wobei die Steuerbefehle vorgeben, wie die Erfassungseinheit 110 arbeitet. Im Falle einer unidirektionalen Datenübertragungsverbindung 114, welche lediglich eine Datenübertragung von der Erfassungseinheit 110 an die Auswerteeinheit 120 ermöglicht, können Steuerparameter über Eingabeelemente (Knöpfe, Schalter, Drehregler, etc., nicht gezeigt) an der Erfassungseinheit 110 vorgegeben werden. Die Erfassungseinheit 110 weist gegebenenfalls Anzeigeelemente (nicht gezeigt) auf, welche einen Status der Erfassungseinheit oder die eingestellten Steuerparameter anzeigen. Alternativ kann die Erfassungseinheit 110 die eingestellten Steuerparameter auch an die Auswerteeinheit 120 übertragen, wo diese wahlweise angezeigt werden können.

Die Auswerteeinheit 120 weist einen Prozessor 126 und einen lokalen Speicher 128 auf. Die Auswerteeinheit 120 empfängt Signale betreffend die Merkmale des untersuchten Bereiches 12 der Haarprobe 10 und ermittelt basierend auf diesen Merkmalen eine Empfehlung betreffend eine nicht-therapeutische Behandlung des untersuchten Haars. Die nicht-therapeutische Behandlung kann Empfehlungen zu Behandlungsmitteln und/oder Behandlungshinweisen oder Anwendungshinweisen für das jeweils untersuchte Haar enthalten. Behandlungshinweise und Anwendungshinweise werden im Kontext dieser Beschreibung als Synonyme verwendet und beziehen sich auf Hinweise für die nicht-therapeutische Behandlung des untersuchten Bereichs (Haars) 12 unter Verwendung von ausgewählten Behandlungsmitteln oder auch ohne eine Verwendung von Behandlungsmitteln. Behandlungshinweise können insbesondere die Anwendung eines Behandlungsmittels enthalten, oder auch von dem Nutzer vorzunehmende oder zu unterlassende Maßnahmen. So können die Behandlungshinweise beispielsweise einen Hinweis auf erwünschtes oder unerwünschtes Verhalten nach der Anwendung eines Behandlungsmittels enthalten. Zum Ermitteln einer zu empfehlenden nicht-therapeutischen Behandlung können die erfassten Merkmale des untersuchten Bereiches 12 mit Anwendungsgebieten, Wirkungen und Anwendungshinweisen von Behandlungsmitteln und/oder Behandlungshinweisen abgeglichen werden. Informationen über die Behandlungsmittel und/oder Behandlungshinweise können in einer Datenspeichereinheit 140 hinterlegt sein.

Die Datenspeichereinheit 140 kann außerhalb und räumlich getrennt von der Auswerteeinheit 120 angeordnet sein. Die Auswerteeinheit 120 kann über ein Datennetz 122 auf die Datenspeichereinheit 140 zugreifen und Informationen über die dort hinterlegten Behandlungsmittel und/oder Behandlungshinweise abrufen. Diese abgerufenen Informationen werden durch die Auswerteeinheit 120 mit den erfassten Merkmalen des zu untersuchenden Bereiches 12 abgeglichen, um zutreffende Empfehlungen für die nicht-therapeutische Behandlung des untersuchten Haars zu ermitteln. In anderen Worten bedeutet das, dass die Datenspeichereinheit unter Verwendung der erfassten Haarmerkmale (oder ermittelten Haareigenschaften) abgefragt wird. Aus der Datenspeichereinheit kann zunächst eine Vielzahl von hinterlegten Informationen abgerufen werden, um diese dann unter Verwendung der ermittelten Haareigenschaften und ggf. von Behandlungszielen danach zu filtern, welche der Behandlungsmittel und/oder Behandlungshinweise einschlägig sind. Hierzu können die Daten aus dem Datenspeicher in einen flüchtigen Arbeitsspeicher geladen werden. Alternativ können aber bereits beim Abrufen der Informationen aus dem Datenspeicher die ermittelten Haareigenschaften herangezogen werden, um nur die einschlägigen Informationen aus dem Datenspeicher abzurufen. Für die Zwecke dieser Beschreibung können diese beiden Varianten als in Ihrer Wirkung gleichwertig verstanden werden.

Das Datennetz 122 kann ein öffentliches Datenübertragungsnetz sein, welches abschnittsweise leitungsgebundene oder leitungslose Übertragungsabschnitte aufweist. Beispielsweise mag die Auswerteeinheit 120 eine drahtlose Verbindung zu einem Zugangspunkt (nicht gezeigt) zu dem Datennetz 122 aufbauen, um eine entsprechende Verbindung zu der Datenspeichereinheit 140 aufbauen zu können.

Die Nutzerschnittstelle 130 ist mit der Auswerteeinheit 120 über die Datenübertragungsverbindung 124 verbunden. Die Nutzerschnittstelle 130 weist eine Eingabeeinheit 132 und eine Ausgabeeinheit 134 auf. Die Eingabeeinheit 132 ermöglicht es einem Nutzer, Parameter für die Arbeitsweise und Konfiguration der Auswerteeinheit 120, der Erfassungseinheit 110 und/oder der Nutzerschnittstelle 130 vorzugeben. Die Eingabeeinheit 132 kann Informationen über verschiedene Schnittstellen aufnehmen: eine Tastatur, eine Maus, ein berührungsempfindliches Display oder über ein Mikrofon (sog. Sprachsteuerung). Es ist denkbar, dass jegliche Schnittstelle genutzt wird, über welche ein menschlicher Nutzer mit einer Recheneinheit kommunizieren und Daten eingeben oder übergeben kann. Über die Eingabeeinheit kann die Luftfeuchtigkeit oder ein Feuchtigkeitsgrad des Haars eingegeben werden. Die Ausgabeeinheit 134 kann ein Display oder eine sonstige Anzeigeeinheit sein, welche einem Nutzer visuelle Informationen ausgibt. Auch kann die Ausgabeeinheit 134 über einen Lautsprecher verfügen, über welchen akustische Informationen ausgegeben werden können. Visuelle Informationen können auf einer berührungsempfindlichen Ausgabeeinheit ausgegeben werden, so dass die Ausgabeeinheit es auch ermöglicht, dass ein Nutzer hierüber Eingaben vornehmen kann.

Die Auswerteeinheit 120 weist einen Prozessor 126 und einen lokalen Speicher 128 auf. Der Prozessor 126 führt Instruktionen aus, um seine vorgesehene Funktion oder Funktionen auszuführen. In dem lokalen Speicher 128 können die von der Erfassungseinheit 110 erfassten Merkmale des Haars oder die zugehörigen Signale oder Werte abgespeichert werden.

Es ist ein besonderer Aspekt dieses Ausführungsbeispiels, dass die Erfassungseinheit 110 mit einer Auswerteeinheit 120 und einer Nutzerschnittstelle 130 betrieben werden können, welche in einem portablen Gerät eines Nutzers oder Verbrauchers implementiert sind. Damit ist es besonders einfach möglich, eine Erfassungseinheit 110, welche fortgeschrittene Analyse- und Untersuchungsmöglichkeiten für das Haar eines menschlichen Nutzers ermöglicht, mit einem tragbaren computerisierten Datenverarbeitungsgerät zu koppeln. Das tragbare Datenverarbeitungsgerät kann beispielsweise ein Smartphone oder ein Tablet sowie ein Heimcomputer sein. Die Erfassungseinheit 110 kann mittels einer definierten Schnittstelle mit dem tragbaren Datenverarbeitungsgerät mechanisch, elektrisch und signaltechnisch verbunden oder gekoppelt werden.

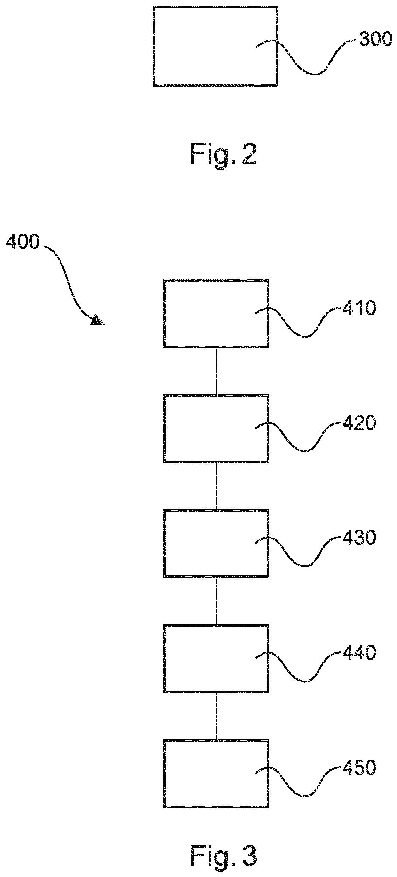

Fig. 2 zeigt einen Datenträger 300. Auf dem Datenträger ist ein Computerprogrammprodukt abgelegt, welches ausgeführt ist, auf einer portablen Recheneinheit 120 ausgeführt zu werden und einen Prozessor 126 der portablen Recheneinheit anzuweisen, die folgenden Schritte auszuführen (vgl. auch die Verfahrensschritte in Fig. 3): Bestrahlen (410) einer Haarprobe mit elektromagnetischen Wellen im Infrarotbereich; Erfassen (420) des von der Haarprobe abgegebenen Lichts; Erfassen (430) eines Absorptionsgrads der Haarprobe in einem Wellenlängenbereich von 800 bis 2500 nm; Generieren (440) eines Absorptionsspektrums der Haarprobe in dem Wellenlängenbereich von 800 bis 2500 nm; Abgleichen (450) des generierten Absorptionsspektrums mit einem Kalibriermodell und Ermitteln des Dehnungsgrades von Haar basierend auf dem Absorptionsspektrum und dem Kalibriermodell.

Der Datenträger 300 kann magnetische, optische oder elektrische Speichertechniken (oder Kombinationen hiervon) nutzen, um die Anweisungen des Computerprogrammprodukts in einer maschinenlesbaren Form vorzuhalten. Diese Anweisungen können verwendet werden, um unmittelbar von dem Prozessor 126 einer portablen Recheneinheit 120 (der Auswerteeinheit 120 aus dem Ausführungsbeispiel der Fig. 1) ausgeführt zu werden. Alternativ können die Anweisungen verwendet werden, um das Computerprogrammprodukt in einen internen Speicher der portablen Recheneinheit 120 zu laden, um dann von dort ausgeführt zu werden. Bei diesem internen Speicher kann es sich beispielsweise um den lokalen Speicher 128 aus der Fig. 1 handeln.

Der Datenträger 300 kann ein mobiler und/oder tragbarer Datenspeicher sein. Alternativ kann das Computerprogrammprodukt auch über ein Datennetz geladen werden, indem von einer portablen Recheneinheit über das Datennetz auf den Datenträger 300 zugegriffen wird, um die Computerprogrammprodukt über das Datennetz zu laden. Das Computerprogrammprodukt kann über ein Datennetz auf ein portables Gerät eines Nutzers geladen und auf dem portablen Gerät zur Verwendung durch den Nutzer installiert werden.

Ergänzend zu Fig. 2 ist in Fig. 3 ein Verfahren 400 mit den folgenden Schritten gezeigt (diese Schritte entsprechen den Funktionen des Computerprogrammprodukts): Bestrahlen (410) einer Haarprobe mit elektromagnetischen Wellen im Infrarotbereich; Erfassen (420) des von der Haarprobe abgegebenen Lichts; Erfassen (430) eines Absorptionsgrads der Haarprobe in einem Wellenlängenbereich von 800 bis 2500 nm; Generieren (440) eines Absorptionsspektrums der Haarprobe in dem Wellenlängenbereich von 800 bis 2500 nm; Abgleichen (450) des generierten Absorptionsspektrums mit einem Kalibriermodell und Ermitteln des Dehnungsgrades von Haar basierend auf dem Absorptionsspektrum und dem Kalibriermodell.

Das Computerprogrammprodukt enthält Anweisungen, welche den Prozessor 126 der portablen Recheneinheit 120 anweisen, diese Schritte 410 bis 450 auszuführen.

Selbstverständlich kann das Verfahren 400 oder dessen Schritte 410 bis 450 in Einklang mit einem der Ausführungsbeispiele der Anordnung 100, wie mit Bezug zu Fig. 1 und der übrigen Beschreibung dargestellt, modifiziert werden. Dies bedeutet, dass die hierin beschriebenen Funktionen der Anordnung 100 oder einer ihrer Komponenten, insbesondere der Auswerteeinheit 120, als Schritt des Verfahrens 400 implementiert werden können. Es wird darauf verzichtet, die Funktionen der Auswerteeinheit an dieser Stelle zu wiederholen. Vielmehr wird der Fachmann erkennen, dass und wie diese Funktionen als Verfahrensschritte implementiert werden.

Die verschiedenen Verfahrensschritte sowie die Komponenten der Anordnung können durch ein oder mehrere Schaltkreise realisiert sein. In einer Ausführungsform ist ein "Schaltkreis" als jegliche Einheit zu verstehen, die eine Logik implementiert, und die sowohl Hardware, Software, Firmware oder eine Kombination daraus sein kann. Somit kann ein "Schaltkreis" in einer Ausführungsform ein hart-verdrahteter Logik-Schaltkreis oder ein programmierbarer Logik-Schaltkreis sein, wie beispielsweise ein programmierbarer Prozessor, z.B. ein Mikroprozessor oder ein FPGA-Baustein (field programmable gate array, programmierbares Logikgatter). Unter einem "Schaltkreis" kann auch ein Prozessor zu verstehen sein, der Software ausführt, z.B. jegliche Art von Computer-Programm, etwa ein Computer-Programm in Programmiercode für eine virtuelle Maschine (abgegrenzte Laufzeitumgebung, Virtual Machine), wie z.B. ein Java-Computer-Programm. Unter einem "Schaltkreis" kann in einer Ausführungsform jegliche Art der Implementierung der im Weiteren beschriebenen Funktionen zu verstehen sein.

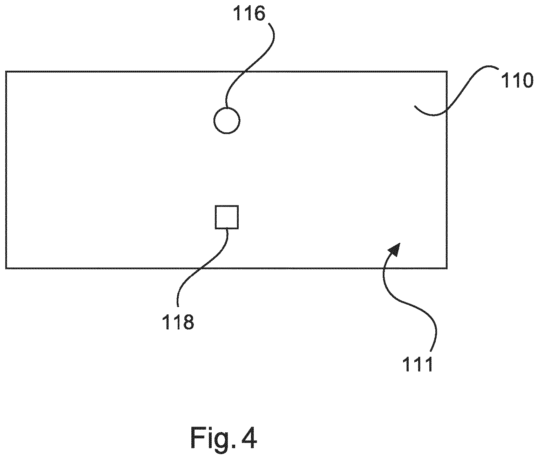

Fig. 4 zeigt eine schematische Darstellung einer Erfassungseinheit 110. Die Erfassungseinheit weist eine Oberfläche 111 auf, an welcher ein Lichtemitter 116 und ein NIR-Sensor 118 gezeigt sind. Der NIR-Sensor 118 kann ein Spektrometer sein. Der Lichtemitter 116 ist kreisförmig dargestellt und der NIR-Sensor 118 ist viereckig dargestellt.

Von der Erfassungseinheit 110 ist diejenige Oberfläche zu sehen, welche bei einem Erfassungsvorgang dem Haar eines Nutzers zugewandt ist. In anderen Worten emittiert der Lichtemitter 116 die Lichtstrahlen aus der Zeichenebene heraus auf einen Betrachter zu.

Wird das Haar eines menschlichen Benutzers mit Licht (z.B. Laser) bestrahlt, wird ein Teil dieses Lichts in Abhängigkeit der chemischen Zusammensetzung und/oder Struktur des Haars abgegeben.

Der Prozessor 126 (Fig. 1) kann Steuerfunktionen implementierten und Steuerbefehle an den Lichtemitter 116 ausgeben. So kann der Prozessor 126 den Lichtemitter ansteuern, um Licht einer gewissen Intensität, Wellenlänge und/oder spektralen Verteilung (diese können als Parameter des Lichts bezeichnet werden) auszugeben.

Die Auswerteeinheit 120 mit dem Prozessor 126 (Fig. 1) empfängt auch die Signale des NIR-Sensors 118 und kann basierend auf diesen Signalen das untersuchte Haar klassifizieren. In anderen Worten sind die von dem NIR-Sensor 118 gelieferten Signale kennzeichnend für das untersuchte Haar. Diese Signale können auch als Signalmuster bezeichnet werden und können genutzt werden, um eine Produktempfehlung und/oder Anwendungshinweise zu ermitteln und auszugeben.

Es ist denkbar, dass einem Produkt und/oder einem Anwendungshinweis ein typisches Signalmuster zugeordnet wird, bei dem das Produkt und oder der Anwendungshinweis sinnvollerweise auf das untersuchte Haar aufgebracht werden kann, um ein gewünschtes Behandlungsergebnis zu erzielen. Dieses zugeordnete Signalmuster der Produkte und/oder der Anwendungshinweise kann mit dem tatsächlichen Signalmuster von der Erfassungseinheit verglichen werden. Ab einem gewissen Grad an Übereinstimmung des von dem Spektrometer erfassten oder gelieferten Signalmusters mit dem den Produkten und/oder Anwendungshinweisen zugeordneten Signalmuster können dann die entsprechenden Produkte und/oder Anwendungshinweise ausgegeben werden. Die Signale können auf qualitative Ähnlichkeit (entsprechen sich die Formen oder Verläufe der Signale) und/oder quantitative Ähnlichkeit (haben die Signale für ähnliche Eingangswerte, d.h. Licht, ähnliche Ausgangswerte, d.h. abgegebenes Licht) untersucht werden.

Es ist auch denkbar, dass in Abhängigkeit von Benutzereingaben ein Faktor ermittelt wird, der auf das von dem Spektrometer erfasste Signal angewendet wird, bevor dieses Eingangssignal mit den Signalmustern der Produkte oder Anwendungshinweisen verglichen wird. Dies hat den Vorteil, dass ein Korrekturfaktor auf das erfasste Signal angewandt werden kann, um die Genauigkeit der Produktempfehlungen und/oder Anwendungshinweise für einen bestimmten Nutzer zu verbessern.

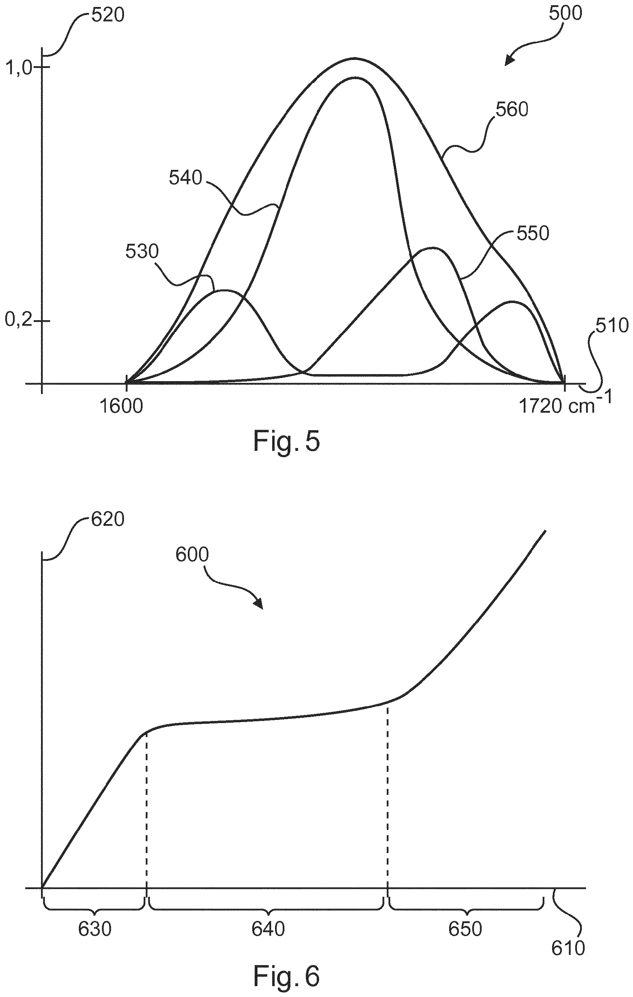

Fig. 5 zeigt ein beispielhaftes Absorptionsdiagramm 500 in einem Wellenzahlbereich von 1600 cm⁻¹ bis 1720 cm⁻¹ (Wellenlängenbereich zwischen 6250 nm und 5813 nm). Der in Fig. 5 gezeigte Wellenlängenbereich befindet sich oberhalb des NIR-Bereichs (nahes Infrarot) und dient dem Verständnis der Erfindung. In dem angezeigten Diagramm wird das Absorptionsspektrum von Haaren in einem Wellenlängenbereich von mittlerem Infrarot gezeigt. Auf der horizontalen Achse 510 ist die Wellenzahl und auf der vertikalen Achse 520 ein Absorptionsgrad gemessen als Anteil des absorbierten Lichts an dem ausgestrahlten Licht (normiert auf 1) aufgetragen. In dem genannten Wellenzahlbereich ist das Absorptionsspektrum 530 der β-Faltblattstrukturen, das Absorptionsspektrum 540 der α-Helices, das Absorptionsspektrum 550 von sonstigen zufällig verteilten Proteinteilstücken des Haars, und die resultierende Gesamtabsorption 560 gezeigt. Die Gesamtabsorption 560 ergibt sich aus der Überlagerung der Kurven 530, 540 und 550. Die qualitative Form und die quantitativen Absorptionswerte der Kurve 560 sind charakteristisch für den Dehnungsgrad der Haarprobe.

Allerdings kann es apparativ sehr aufwändig sein, das Absorptionsspektrum oberhalb des NIR-Bereichs (oberhalb von einer Wellenlänge von 3000 nm) zu erfassen. Die Erfindung schlägt daher vor, einen Nahinfrarotsensor zu verwenden, welcher einen Absorptionsgrad einer Haarprobe in einem Wellenlängenbereich zwischen 800 nm und 2500 nm erfasst und das zugehörige Absorptionsspektrum generiert. In dem Wellenlängenbereich zwischen 800 nm und 2500 nm, insbesondere zwischen 2000 und 2500 nm, können Oberschwingungen der α-Helices und β-Faltblattstrukturen erfasst werden.

Fig. 6 zeigt beispielhaft und qualitativ ein Spannungs-Dehnungs-Diagramm 600 für menschliches Haar. Auf der horizontalen Achse 610 ist die Dehnung und auf der vertikalen Achse 620 die in dem Haar herrschende Spannung aufgetragen.

Bei menschlichem Haar verläuft der Dehnungsvorgang bis ca. 3% bis 5 % Dehnung (in Abhängigkeit von dem individuellen Haar) vollständig reversibel. Das bedeutet, dass die Haare nach Entlastung nach kurzer Zeit ihre Ausgangslänge vor der Dehnung wieder einnehmen. Dieser Bereich ist mit 630 gekennzeichnet und gibt den Bereich linear-elastischen Verhaltens an. Ab ca. 10% bis 15 % Dehnung erfolgt die irreversible Umwandlung von α-Helices in β-Faltblattstrukturen. Diese Überdehnung erfordert aufgrund der einfachen Umwandlung der Sekundärstrukturen nur recht geringe Kräfte, wie man an einer sehr geringen oder kaum vorhandenen Steigung der Kurve in dem Bereich 640 erkennen kann. Bei ca. 25% Dehnung reißt das Haar.

Diese Umwandlung des Haares ist jedoch aus kosmetischer Sicht unerwünscht, da das Haar in dem Übergangsbereich 640 seine mechanische Stabilität deutlich einbüßt.

Anschließend an den Bereich 640 gibt es noch einen weiteren Bereich 650, welcher einer weiter zunehmenden Dehnung entspricht. Hier steigt die Spannung mit zunehmender Dehnung weiter an, bis das Haar letztlich reißt.

Die Analyse des Verhältnisses von α-Helices zu β-Faltblattstrukturen ist daher neben Parametern wie oxidativer Schaden, reduktiver Schaden, Feuchtigkeit, Oberflächenschaden eine weitere wichtige Größe zur holistischen Erfassung des Haarzustandes.

Es wurde nun gefunden, dass sich die beschriebene Überdehnung von Haaren mit Hilfe von NIR-Sensoren einfach ermitteln lässt, indem das mit dem NIR-Sensor erfasste Absorptionsspektrum mit den Absorptionsspektren eines Kalibriermodells verglichen wird, wie oben beschrieben. Aufgrund der Kompaktheit und der geringen Kosten ist das Verfahren dabei auch für Endverbraucher, Friseursalons und Drogeriemärkte ("Point of Sale") geeignet.

### LISTE DER BEZUGSZEICHEN

- 10: Analyseobjekt
- 12: zu untersuchender Bereich
- 100: Anordnung zum Ermitteln von Eigenschaften von Haar
- 110: Erfassungseinheit
- 111: Oberfläche
- 112: elektromagnetische Wellen
- 114: Datenübertragungsverbindung
- 116: Lichtemitter
- 118: NIR-Sensor
- 120: Auswerteeinheit
- 122: Datennetz
- 124: Datenübertragungsverbindung
- 126: Prozessor
- 128: lokaler Speicher
- 130: Nutzerschnittstelle
- 132: Eingabeeinheit
- 134: Ausgabeeinheit
- 140: Datenspeichereinheit
- 200: Gehäuse
- 210: Energiespeicher
- 300: Datenträger
- 400: Verfahren
- 410 -: 450 Verfahrensschritte
- 500: Absorptionsdiagramm
- 510: Wellenzahl
- 520: Absorption
- 530: β-Faltblattstrukturen
- 540: α-Helices
- 550: sonstige Proteinteilstücke
- 560: Gesamtabsorption
- 600: Spannungs-Dehnungs-Diagramm
- 610: Dehnung
- 620: Spannung
- 630: linear-elastisches Verhalten, reversibler Bereich
- 640: Umwandlungsbereich von α-Helices in β-Faltstrukturen
- 650: Überdehnung

## Patentansprüche

1. Anordnung (100) zum Bestimmen eines Dehnungsgrades von Haar, die Anordnung aufweisend:
eine Erfassungseinheit (110) zum Erfassen von Haarmerkmalen;
eine Auswerteeinheit (120) zum Auswerten der erfassten Haarmerkmale und zum Ermitteln des Dehnungsgrades von Haar basierend auf den erfassten Haarmerkmalen;
wobei die Erfassungseinheit (110) einen Nahinfrarotsensor, NIRS, enthält;
wobei die Erfassungseinheit (110) ausgeführt ist, eine Haarprobe mit elektromagnetischen Wellen im Infrarotbereich zu bestrahlen und einen Absorptionsgrad der Haarprobe in einem Wellenlängenbereich von 800 bis 2500 nm zu erfassen;
wobei die Erfassungseinheit (110) ausgeführt ist, ein Absorptionsspektrum der Haarprobe in dem Wellenlängenbereich von 800 bis 2500 nm zu generieren und der Auswerteeinheit (120) bereitzustellen;
wobei die Auswerteeinheit (120) ausgeführt ist, das generierte Absorptionsspektrum mit einem Kalibriermodell abzugleichen und einen Dehnungsgrad der Haarprobe basierend auf dem Absorptionsspektrum und dem Kalibriermodell zu ermitteln.

2. Anordnung (100) nach Anspruch 1,
wobei die Auswerteeinheit (120) ausgeführt ist, einen Verlauf des Absorptionsspektrums der Haarprobe mit einer Mehrzahl von Absorptionsspektren aus dem Kalibriermodell abzugleichen.

3. Anordnung (100) nach Anspruch 1 oder 2,
wobei die Erfassungseinheit (110) ausgeführt ist, einen Feuchtigkeitsgrad der Haarprobe zu erfassen oder zu erhalten und den Feuchtigkeitsgrad der Auswerteeinheit (120) bereitzustellen;
wobei die Auswerteeinheit (120) ausgeführt ist, den Feuchtigkeitsgrad der Haarprobe beim Abgleich des generierten Absorptionsspektrums mit dem Kalibriermodell zu berücksichtigen.

4. Anordnung (100) nach einem der voranstehenden Ansprüchen,
wobei die Auswerteeinheit (120) ausgeführt ist, der Haarprobe basierend auf dem Abgleich des generierten Absorptionsspektrums mit dem Kalibriermodell eine Schadenskategorie zuzuordnen.

5. Anordnung (100) nach einem der voranstehenden Ansprüche,
wobei der Nahinfrarotsensor ein Spektrometer (118) ist, wobei das Spektrometer ausgeführt ist, basierend auf dem erfassten Licht ein Signalmuster zu erstellen, welches charakteristisch für die erfassten Haarmerkmale ist.

6. Anordnung (100) nach einem der voranstehenden Ansprüche,
weiterhin aufweisend ein Gehäuse (200) und einen Energiespeicher (210), wobei die Auswerteeinheit (120) in dem Gehäuse untergebracht und die Erfassungseinheit (110) mit dem Gehäuse gekoppelt ist, und wobei der Energiespeicher in dem Gehäuse (200) angeordnet ist, um die Auswerteeinheit (120) mit Energie zu versorgen, und um zumindest zeitweise einen autarken Betrieb der Auswerteeinheit ohne Anschluss an eine externe Energiequelle zu ermöglichen.

7. Anordnung (100) nach einem der voranstehenden Ansprüche,
wobei die Auswerteeinheit (120) ausgeführt ist, die Merkmale von Behandlungsmitteln zur Behandlung von Haar mit den erfassten Haarmerkmalen abzugleichen und eine Wirkung der Behandlungsmittel auf die Haare unter Berücksichtigung der erfassten Haarmerkmale zu bestimmen.

8. Anordnung (100) nach einem der voranstehenden Ansprüche,
wobei die Auswerteeinheit (120) ausgeführt ist, die erfassten Haarmerkmale an eine Datenspeichereinheit (140) zu übertragen und Hinweise zur Behandlung des Haars gemäß den erfassten Haarmerkmalen von der Datenspeichereinheit abzufragen.

9. Anordnung (100) nach Anspruch 8,
weiterhin aufweisend eine Nutzerschnittstelle (130),
wobei die Auswerteeinheit (120) ausgeführt ist, die Nutzerschnittstelle anzuweisen, die erhaltenen Hinweise zur Behandlung der Haare auszugeben.

10. Anordnung (100) nach einem der Ansprüche 8 oder 9,
wobei die Auswerteeinheit (120) ausgeführt ist, Informationen von einem Nutzer abzufragen und diese Informationen beim Abfragen der Datenspeichereinheit (140) zusätzlich zu berücksichtigen, um von der Datenspeichereinheit (140) Merkmale von Behandlungsmitteln zur Behandlung von Haar gemäß der von dem Nutzer abgefragten Informationen zu erhalten.

11. Verfahren (400) zum Bestimmen eines Dehnungsgrades von Haar, aufweisend:
Bestrahlen (410) einer Haarprobe mit elektromagnetischen Wellen im Infrarotbereich;
Erfassen (420) des von der Haarprobe abgegebenen Lichts;
Erfassen (430) eines Absorptionsgrads der Haarprobe in einem Wellenlängenbereich von 800 bis 2500 nm;
Generieren (440) eines Absorptionsspektrums der Haarprobe in dem Wellenlängenbereich von 800 bis 2500 nm;
Abgleichen (450) des generierten Absorptionsspektrums mit einem Kalibriermodell und Ermitteln des Dehnungsgrades von Haar basierend auf dem Absorptionsspektrum und dem Kalibriermodell.

12. Verfahren (400) nach Anspruch 11, weiter aufweisend:
Erfassen oder Erhalten eines Feuchtigkeitsgrads der Haarprobe; und
Heranziehen des Feuchtigkeitsgrads der Haarprobe beim Abgleich des generierten Absorptionsspektrums mit dem Kalibriermodell.

13. Verfahren (400) nach einem der Ansprüche 11 oder 12, weiter aufweisend:
Zuordnen einer Schadenskategorie zu der Haarprobe basierend auf dem Abgleich des generierten Absorptionsspektrums mit dem Kalibriermodell.

14. Verfahren zum Ermitteln eines Behandlungsmittels basierend auf dem in einem der Ansprüche 11 bis 13 bestimmten Dehnungsgrad von Haar, aufweisend die folgenden Schritte:
Heranziehen des bestimmten Dehnungsgrades von Haar;
Auswählen eines Behandlungsmittels für Haar basierend auf dem ermittelten Dehnungsgrad und Ausgeben von Informationen über das ausgewählte Behandlungsmittel.

15. Computerprogrammprodukt, welches ausgeführt ist, das Verfahren nach einem der Ansprüche 11 bis 14 auszuführen, wenn es auf einer Anordnung nach einem der Ansprüche 1 bis 10 ausgeführt wird.

## Claims

1. An arrangement (100) for determining a degree of elongation of hair, the arrangement comprising:
a detection unit (110) for detecting hair characteristics;
an evaluation unit (120) for evaluating the detected hair characteristics and for determining the degree of elongation of hair based on the detected hair characteristics;
wherein the detection unit (110) contains a near-infrared sensor, NIRS;
wherein the detection unit (110) is designed to irradiate a hair sample with electromagnetic waves in the infrared range and to detect an absorbance of the hair sample in a wavelength range of 800 to 2500 nm;
wherein the detection unit (110) is designed to generate an absorption spectrum of the hair sample in the wavelength range of 800 to 2500 nm and to provide the absorption spectrum to the evaluation unit (120);
wherein the evaluation unit (120) is designed to compare the generated absorption spectrum with a calibration model and to determine a degree of elongation of the hair sample based on the absorption spectrum and the calibration model.

2. The arrangement (100) according to claim 1,
wherein the evaluation unit (120) is designed to compare a course of the absorption spectrum of the hair sample with a plurality of absorption spectra from the calibration model.

3. The arrangement (100) according to claim 1 or 2,
wherein the detection unit (110) is designed to detect or obtain a moisture level of the hair sample and to provide the moisture level to the evaluation unit (120);
wherein the evaluation unit (120) is designed to take into account the moisture level of the hair sample when comparing the generated absorption spectrum with the calibration model.

4. The arrangement (100) according to one of the preceding claims,
wherein the evaluation unit (120) is designed to assign a damage category to the hair sample based on the comparison of the generated absorption spectrum with the calibration model.

5. The arrangement (100) according to one of the preceding claims,
wherein the near-infrared sensor is a spectrometer (118), wherein the spectrometer is designed to create a signal pattern based on the detected light, which pattern is characteristic of the detected hair characteristics.

6. The arrangement (100) according to one of the preceding claims,
further comprising a housing (200) and an energy store (210), wherein the evaluation unit (120) is accommodated in the housing and the detection unit (110) is coupled to the housing, and wherein the energy store is arranged in the housing (200) in order to supply the evaluation unit (120) with energy, and to allow at least temporarily autonomous operation of the evaluation unit without said unit being connected to an external energy source.

7. The arrangement (100) according to one of the preceding claims,
wherein the evaluation unit (120) is designed to compare the characteristics of treatment agents for treating hair with the detected hair characteristics and to determine an effect of the treatment agents on the hair, taking into account the detected hair characteristics.

8. The arrangement (100) according to one of the preceding claims,
wherein the evaluation unit (120) is designed to transmit the detected hair characteristics to a data storage unit (140) and to request information for treating the hair according to the detected hair characteristics from the data storage unit.

9. The arrangement (100) according to claim 8,
further comprising a user interface (130),
wherein the evaluation unit (120) is designed to instruct the user interface to output the obtained information for treating the hair.

10. The arrangement (100) according to one of claims 8 or 9,
wherein the evaluation unit (120) is designed to request information from a user and to also take this information into account when requesting information from the data storage unit (140) in order to obtain, from the data storage unit (140), characteristics of treatment agents for treating hair according to the information requested from the user.

11. A method (400) for determining a degree of elongation of hair, comprising:
irradiating (410) a hair sample with electromagnetic waves in the infrared range;
detecting (420) the light emitted by the hair sample;
detecting (430) an absorbance of the hair sample in a wavelength range of 800 to 2500 nm;
generating (440) an absorption spectrum of the hair sample in the wavelength range of 800 to 2500 nm;
comparing (450) the generated absorption spectrum with a calibration model and determining the degree of elongation of hair based on the absorption spectrum and the calibration model.

12. The method (400) according to claim 11, further comprising:
detecting or obtaining a moisture level of the hair sample; and
referring to the moisture level of the hair sample when comparing the generated absorption spectrum with the calibration model.

13. The method (400) according to one of claims 11 or 12, further comprising:
assigning a damage category to the hair sample based on the comparison of the generated absorption spectrum with the calibration model.

14. A method for determining a treatment agent based on the degree of elongation of hair determined in one of claims 11 to 13, comprising the following steps:
referring to the determined degree of elongation of hair;
selecting a treatment agent for hair based on the determined degree of elongation and outputting information about the selected treatment agent.

15. A computer program product which is designed to carry out the method according to one of claims 11 to 14 when said method is carried out on an arrangement according to one of claims 1 to 10.

## Revendications

1. Système (100) permettant de définir un degré d'allongement de cheveux, le système présentant :
une unité de détection (110) permettant de détecter des propriétés de cheveux ;
une unité d'évaluation (120) permettant d'évaluer les propriétés de cheveux détectées et de déterminer le degré d'allongement des cheveux sur la base des propriétés de cheveux détectées ;
dans lequel l'unité de détection (110) contient un capteur proche infrarouge, NIRS ;
dans lequel l'unité de détection (110) est configurée pour irradier un échantillon de cheveux avec des ondes électromagnétiques dans le domaine infrarouge et pour détecter un degré d'absorption de l'échantillon de cheveux dans une gamme de longueurs d'onde allant de 800 à 2500 nm ;
dans lequel l'unité de détection (110) est configurée pour générer un spectre d'absorption de l'échantillon de cheveux dans la gamme de longueurs d'onde allant de 800 à 2500 nm et pour le fournir à l'unité d'évaluation (120) ;
dans lequel l'unité d'évaluation (120) est configurée pour comparer le spectre d'absorption généré avec un modèle d'étalonnage et pour déterminer un degré d'allongement de l'échantillon de cheveux sur la base du spectre d'absorption et du modèle d'étalonnage.

2. Système (100) selon la revendication 1,
dans lequel l'unité d'évaluation (120) est configurée pour comparer une évolution du spectre d'absorption de l'échantillon de cheveux avec une pluralité de spectres d'absorption provenant du modèle d'étalonnage.

3. Système (100) selon la revendication 1 ou 2,
dans lequel l'unité de détection (110) est configurée pour détecter ou obtenir un degré d'humidité de l'échantillon de cheveux et pour fournir le degré d'humidité à l'unité d'évaluation (120) ;
dans lequel l'unité d'évaluation (120) est configurée pour prendre en compte le degré d'humidité de l'échantillon de cheveux lors de la comparaison du spectre d'absorption généré avec le modèle d'étalonnage.

4. Système (100) selon l'une des revendications précédentes,
dans lequel l'unité d'évaluation (120) est configurée pour attribuer une catégorie d'endommagement à l'échantillon de cheveux sur la base de la comparaison du spectre d'absorption généré avec le modèle d'étalonnage.

5. Système (100) selon l'une des revendications précédentes,
dans lequel le capteur proche infrarouge est un spectromètre (118), dans lequel le spectromètre est configuré pour créer un modèle de signal sur la base de la lumière détectée, lequel modèle de signal est caractéristique des propriétés de cheveux détectées.

6. Système (100) selon l'une des revendications précédentes,
présentant en outre un boîtier (200) et un accumulateur d'énergie (210), dans lequel l'unité d'évaluation (120) est logée dans le boîtier et l'unité de détection (110) est accouplée au boîtier, et dans lequel l'accumulateur d'énergie est disposé dans le boîtier (200) afin d'alimenter en énergie l'unité d'évaluation (120) et afin de permettre au moins temporairement un fonctionnement autarcique de l'unité d'évaluation sans connexion à une source d'énergie externe.

7. Système (100) selon l'une des revendications précédentes,
dans lequel l'unité d'évaluation (120) est configurée pour comparer les propriétés d'agents de traitement pour le traitement des cheveux avec les propriétés de cheveux détectées et pour définir un effet des agents de traitement sur les cheveux en prenant en compte les propriétés de cheveux détectées.

8. Système (100) selon l'une des revendications précédentes,
dans lequel l'unité d'évaluation (120) est configurée pour transmettre les propriétés de cheveux détectées à une unité de stockage de données (140) et pour demander des indications concernant le traitement des cheveux conformément aux propriétés de cheveux détectées à l'unité de stockage de données.

9. Système (100) selon la revendication 8,
présentant en outre une interface utilisateur (130),
dans lequel l'unité d'évaluation (120) est configurée pour instruire à l'interface utilisateur de distribuer les indications obtenues concernant le traitement des cheveux.

10. Système (100) selon l'une des revendications 8 ou 9,
dans lequel l'unité d'évaluation (120) est configurée pour demander des informations à un utilisateur et également pour prendre en compte lesdites informations lors de la demande à l'unité de stockage de données (140) afin d'obtenir de l'unité de stockage de données (140) des propriétés d'agents de traitement pour le traitement des cheveux conformément aux informations demandées à l'utilisateur.

11. Procédé (400) permettant de définir un degré d'allongement des cheveux, présentant :
l'irradiation (410) d'un échantillon de cheveux avec des ondes électromagnétiques dans le domaine infrarouge ;
la détection (420) de la lumière émise par l'échantillon de cheveux ;
la détection (430) d'un degré d'absorption de l'échantillon de cheveux dans une gamme de longueurs d'onde allant de 800 à 2500 nm ;
la génération (440) d'un spectre d'absorption de l'échantillon de cheveux dans la gamme de longueurs d'onde allant de 800 à 2500 nm ;
la comparaison (450) du spectre d'absorption généré avec un modèle d'étalonnage et la détermination du degré d'allongement des cheveux sur la base du spectre d'absorption et du modèle d'étalonnage.

12. Procédé (400) selon la revendication 11, présentant en outre :
la détection ou l'obtention d'un degré d'humidité de l'échantillon de cheveux ; et
l'utilisation du degré d'humidité de l'échantillon de cheveux lors de la comparaison du spectre d'absorption généré avec le modèle d'étalonnage.

13. Procédé (400) selon l'une des revendications 11 ou 12, présentant en outre :
l'attribution d'une catégorie d'endommagement à l'échantillon de cheveux sur la base de la comparaison du spectre d'absorption généré avec le modèle d'étalonnage.

14. Procédé permettant de déterminer un agent de traitement sur la base du degré d'allongement des cheveux défini dans l'une des revendications 11 à 13, présentant les étapes suivantes :
utilisation du degré d'allongement défini des cheveux ;
sélection d'un agent de traitement pour les cheveux sur la base du degré d'allongement déterminé et distribution d'informations concernant l'agent de traitement sélectionné.

15. Produit-programme informatique qui est configuré pour exécuter le procédé selon l'une des revendications 11 à 14 lorsqu'il est exécuté sur un système selon l'une des revendications 1 à 10.
